# EUROPÄISCHE PATENTSCHRIFT

(11) **EP 0 828 738 B1**
(45) Veröffentlichungstag und Bekanntmachung des Hinweises auf die Patenterteilung: **17.12.2003**
(21) Anmeldenummer: 96916062.1
(22) Anmeldetag: 14.05.1996
(51) Int. Cl.: C07D 417/04, A01N 43/78, C07D 213/61, A01N 43/40

(54) **PYRIDYL-THIAZOLE UND DEREN VERWENDUNG ZUM SCHUTZ VON PFLANZEN GEGEN BEFALL DURCH MIKROORGANISMEN**
PYRIDYL-THIAZOLES AND THEIR USE TO PROTECT PLANTS AGAINST INFECTIONS BY MICRO-ORGANISMS
PYRIDYL-THIAZOLES ET LEUR UTILISATION POUR PROTEGER DES PLANTES CONTRE DES INFECTIONS PAR DES MICRO-ORGANISMES

(30) Priorität: 26.05.1995 DE 19519332; 04.03.1996 DE 19608244
(43) Veröffentlichungstag der Anmeldung: 18.03.1998
(73) Patentinhaber: Bayer CropScience AG, 40789 Monheim (DE)
(72) Erfinder: GALLENKAMP, Bernd, D-42113 Wuppertal (DE); STENZEL, Klaus, D-40595 Düsseldorf (DE); HÄNSSLER, Gerd, D-51381 Leverkusen (DE); DUTZMANN, Stefan, D-40721 Hilden (DE); DEHNE, Heinz-Wilhelm, D-53125 Bonn (DE)
(86) Internationale Anmeldenummer: EP9602052
(87) Internationale Veröffentlichungsnummer: WO96037493

(56) Entgegenhaltungen:
- EP-A- 0 169 502
- EP-A- 0 268 775
- WO-A-93/10095

## Beschreibung

Die vorliegende Erfindung betrifft Pyridyl-thiazole, mehrere Verfahren zu deren Herstellung und deren Verwendung zum Schutz von Pflanzen gegen Befall durch unerwünschte Mikroorganismen.

Es ist bereits bekannt geworden, daß sich bestimmte Halogenpyridin-4-carbonsäure-Derivate zur Erzeugung von Resistenz in Pflanzen gegen Befall durch phytopathogene Mikroorganismen einsetzen lassen (vgl. EP-OS 0 268 775 und DE-OS 4 138 026). So können z.B. 2,6-Dichlorpyridin-4-carbonsäure, 2,6-Dichlorpyridin-4-carbonsäure-methylester und 2,6-Dichlorpyridin-4-carbonsäure-α-(4-chlorphenyl)-benzylester für den genannten Zweck verwendet werden. Die Wirkung dieser Stoffe ist aber vor allem bei niedrigen Aufwandmengen nicht immer befriedigend. Außerdem läßt die direkte fungizide Wirkung dieser Verbindungen zu wünschen übrig.

Weiterhin sind aus der EP - A 0 169 502 2-Benzyl- 4 - (4 - pyridyl) - thiazole mit immunoregulierender Wirksamkeit bekannt. Entsprechende Verbindungen, in denen der Thiazolring in der 2 - Stellung durch andere Reste als Benzyl substituiert ist, werden allerdings nicht offenbart. Außerdem wird von den beschriebenen Stoffen keine Verwendung gegen unerwünschte Mikroorganismen erwähnt.

Es wurden nun Pyridyl-thiazole der Formel 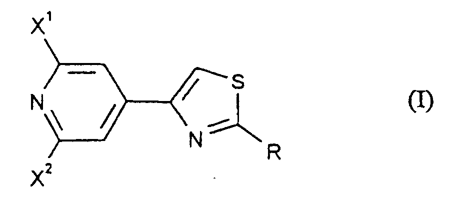 in welcher
- R: für geradkettiges oder verzweigtes Alkyl mit 1 bis 6 Kohlenstoffatomen sowie für die Gruppen 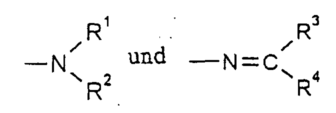 steht, worin
- R¹: für Wasserstoff oder -CO-R⁵ steht,
- R²: für Wasserstoff oder -CO-R⁶ steht,
- R³: für Wasserstoff oder geradkettiges oder verzweigtes Alkyl mit 1 bis 4 Kohlenstoffatomen steht,
- R⁴: für Alkoxy mit 1 bis 4 Kohlenstoffatomen oder für Dialkylamino mit 1 bis 4 Kohlenstoffatomen in jeder Alkylgruppe steht und
- R⁵ und R⁶: unabhängig voneinander für geradkettiges oder verzweigtes Alkyl mit 1 bis 6 Kohlenstoffatomen, Alkoxycarbonyl mit 1 bis 4 Kohlenstoffatomen in der Alkoxygruppe, Alkylamino mit 1 bis 6 Kohlenstoffatomen, Aryl mit 6 bis 10 Kohlenstoffatomen, Arylamino mit 6 bis 10 Kohlenstoffatomen oder Arylsulfonylamino mit 6 bis 10 Kohlenstoffatomen stehen, wobei jeder der drei zuletzt genannten Reste im Arylteil einfach bis dreifach, gleichartig oder verschieden substituiert sein kann durch Halogen, Cyano, Nitro, Formyl, Carboxyl, Carbamoyl, Alkyl mit 1 bis 6 Kohlenstoffatomen, Alkoxy mit 1 bis 6 Kohlenstoffatomen, Alkylthio mit 1 bis 6 Kohlenstoffatomen, Alkylsulfinyl mit 1 bis 4 Kohlenstoffatomen, Alkylsulfonyl mit 1 bis 4 Kohlenstoffatomen, Halogenalkyl mit 1 bis 4 Kohlenstoffatomen und 1 bis 5 Fluor-, Chlor- und/oder Bromatomen, Halogenalkoxy mit 1 bis 4 Kohlenstoffatomen und 1 bis 5 Fluor-, Chlor- und/oder Bromatomen, Halogenalkylthio mit 1 bis 4 Kohlenstoffatomen und 1 bis 5 Fluor-, Chlor- und/oder Bromatomen, Halogenalkylsulfinyl mit 1 bis 4 Kohlenstoffatomen und 1 bis 5 Fluor-, Chlor- und/oder Bromatomen, Halogenalkylsulfonyl mit 1 bis 4 Kohlenstoffatomen und 1 bis 5 Fluor-, Chlor- und/oder Bromatomen, Alkoxycarbonyl mit 1 bis 4 Kohlenstoffatomen im Alkoxyteil, Hydroximinoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil und/oder durch zweifach verknüpftes Alkandiyl mit 3 oder 4 Kohlenstoffatomen, in dem ein oder zwei (nicht benachbarte) Kohlenstoffatome durch Sauerstoff ersetzt sein können und in dem der Alkandiylteil einfach bis vierfach, gleichartig oder verschieden substituiert sein kann durch Halogen, Alkyl mit 1 bis 4 Kohlenstoffatomen und/oder Halogenalkyl mit 1 oder 2 Kohlenstoffatomen und 1 bis 3 Fluor-, Chlor- und/oder Bromatomen,
oder
- R¹ und R²: gemeinsam mit dem Stickstoffatom, an das sie gebunden sind, für ein cyclisches Imid der Formel 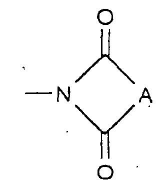 stehen, worin
A für Alkandiyl mit 2 oder 3 Kohlenstoffatomen oder Alkendiyl mit 2 oder 3 Kohlenstoffatomen steht, wobei die genannten Reste jeweils einfach bis vierfach, gleichartig oder verschieden substituiert sein können durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Fluor-, Chlor- und/oder Bromatomen und/oder durch Halogenalkoxy mit 1 bis 4 Kohlenstoffatomen und 1 bis 5 Fluor-, Chlor- und/oder Bromatomen,
- X¹: für Wasserstoff, Fluor, Chlor oder Brom steht
und
- X²: für Fluor, Chlor oder Brom steht,
sowie deren Salze und Säureaddukte gefunden.

Weiterhin wurde gefunden, daß man Pyridyl-thiazole der Formel (I) sowie deren Salze und Säureaddukte erhält, wenn man
a) Halogenacetyl-pyridine der Formel 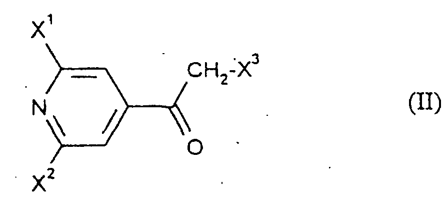 in welcher
   - X¹ und X²: die oben angegebenen Bedeutungen haben und
   - X³: für Halogen steht,
   mit Thioverbindungen der Formel 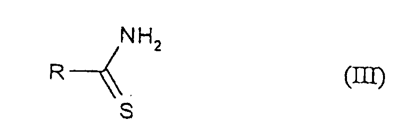 in welcher
   - R: die oben angegebene Bedeutung hat,
   gegebenenfalls in Gegenwart eines Verdünnungsmittels und gegebenenfalls in Gegenwart eines Säurebindemittels umsetzt,
   oder
b) Aminothiazole der Formel 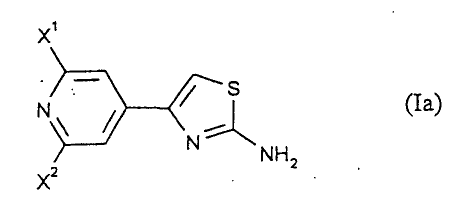 in welcher
   - X¹ und X²: die oben angegebenen Bedeutungen haben,
   entweder
α) mit Carbonsäureanhydriden der Formeln 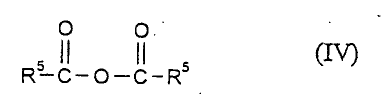 in welcher
   - R⁵: die oben angegebene Bedeutung hat,
   oder 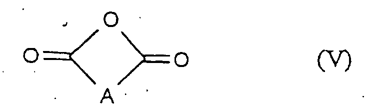 in welcher
   - A: die oben angegebene Bedeutung hat,
   oder
β) mit Carbonsäurehalogeniden der Formel 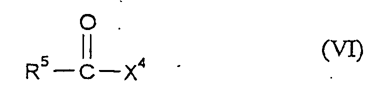 in welcher
   - R⁵: die oben angegebene Bedeutung hat und
   - X⁴: für Halogen steht,
   oder
γ) mit Isocyanaten der Formel

   R⁷-N=C=O (VII)

   in welcher
   R⁷ für geradkettiges oder verzweigtes Alkyl mit 1 bis 6 Kohlenstoffatomen, Aryl mit 6 bis 10 Kohlenstoffatomen oder Arylsulfonyl mit 6 bis 10 Kohlenstoffatomen steht,
   wobei jeder der zwei zuletzt genannten Reste im Arylteil einfach bis dreifach, gleichartig oder verschieden substituiert sein kann durch Halogen, Cyano, Nitro, Formyl, Carboxyl, Carbamoyl, Alkyl mit 1 bis 6 Kohlenstoffatomen, Alkoxy mit 1 bis 6 Kohlenstoffatomen, Alkylthio mit 1 bis 6 Kohlenstoffatomen, Alkylsulfinyl mit 1 bis 4 Kohlenstoffatomen, Alkylsulfonyl mit 1 bis 4 Kohlenstoffatomen, Halogenalkyl mit 1 bis 4 Kohlenstoffatomen und 1 bis 5 Fluor-, Chlor- und/oder Bromatomen, Halogenalkoxy mit 1 bis 4 Kohlenstoffatomen und 1 bis 5 Fluor-, Chlor- und/oder Bromatomen, Halogenalkylthio mit 1 bis 4 Kohlenstoffatomen und 1 bis 5 Fluor-, Chlor- und/oder Bromatomen, Halogenalkylsulfinyl mit 1 bis 4 Kohlenstoffatomen und 1 bis 5 Fluor-, Chlor- und/oder Bromatomen, Halogenalkylsulfonyl mit 1 bis 4 Kohlenstoffatomen und 1 bis 5 Fluor-, Chlor- und/oder Bromatomen, Alkoxycarbonyl mit 1 bis 4 Kohlenstoffatomen im Alkoxyteil, Hydroximinoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil und/oder durch zweifach verknüpftes Alkandiyl mit 3 oder 4 Kohlenstoffatomen, in dem ein oder zwei (nicht benachbarte) Kohlenstoffatome durch Sauerstoff ersetzt sein können und in dem der Alkandiylteil einfach bis vierfach, gleichartig oder verschieden substituiert sein kann durch Halogen, Alkyl mit 1 bis 4 Kohlenstoffatomen und/oder Halogenalkyl mit 1 oder 2 Kohlenstoffatomen und 1 bis 3 Fluor-, Chlor-, und/oder Bromatomen,
   oder
δ) mit Acetalen der Formel 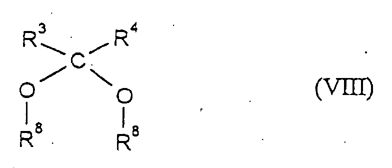 in welcher
   - R³ und R⁴: die oben angegebenen Bedeutungen haben und
   - R⁸: für Methyl oder Ethyl steht,
   gegebenenfalls in Gegenwart eines Verdünnungsmittels, gegebenenfalls eines Katalysators und gegebenenfalls in Gegenwart eines Säurebindemittels umsetzt,
   und gegebenenfalls anschließend die so erhaltenen Verbindungen der Formel (I) mit einer Säure oder Base umsetzt.

Schließlich wurde gefunden, daß die Pyridyl-thiazole der Formel (I) sowie deren Salze und Säureaddukte sehr gut zum Schutz von Pflanzen gegen Befall durch unerwünschte Mikroorganismen verwendbar sind. Die erfindungsgemäßen Stoffe eignen sich sowohl zur Erzeugung von Resistenz in Pflanzen gegen Befall durch unerwünschte Mikroorganismen als auch als Mikrobizide zur direkten Bekämpfung der Mikroorganismen.

Überraschenderweise eignen sich die erfindungsgemäßen Stoffe besser zur Erzeugung von Resistenz in Pflanzen gegen Befall durch phytopathogene Mikroorganismen als die 2,6-Dichlor-pyridin-4-carbonsäure, der 2,6-Dichlor-pyridin-4-carbonsäuremethylester und der 2,6-Dichlor-pyridin-4-carbonsäure-α-(4-chlorphenyl)-benzylester, welches konstitutionell ähnliche, aus dem Stand der Technik vorbekannte Verbindungen gleicher Wirkungsrichtung sind. Außerdem übertreffen die erfindungsgemäßen Stoffe überraschenderweise auch die strukturell ähnlichsten, vorbeschriebenen Substanzen bezüglich ihrer fungiziden Wirksamkeit.

Die erfindungsgemäßen Stoffe können gegebenenfalls als Mischungen verschiedener möglicher isomerer Formen, wie z.B. in Form von Stereoisomeren oder Tautomeren; vorliegen. Die Erfindung betrifft sowohl Stereoisomere als auch Tautomere und auch beliebige Mischungen dieser Isomeren.

Die erfindungsgemäßen Pyridyl-thiazole sind durch die Formel (I) allgemein definiert.
- R: steht bevorzugt für Methyl, Ethyl, n-Propyl, Isopropyl, n-Butyl, sek.-Butyl, iso-Butyl, tert.-Butyl sowie für die Gruppen 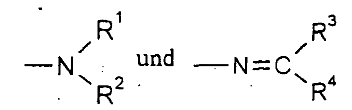
R¹ steht auch bevorzugt für Wasserstoff oder -CO-R⁵.
R² steht auch bevorzugt für Wasserstoff oder -CO-R⁶.
R³ steht bevorzugt für Wasserstoff, Methyl oder Ethyl.
R⁴ steht bevorzugt für Methoxy, Ethoxy, Dimethylamino oder Diethylamino.
R⁵ und R⁶ stehen unabhängig voneinander bevorzugt für Methyl, Ethyl, n-Propyl, Isopropyl, n-Butyl, sek.-Butyl, iso-Butyl, tert.-Butyl, Methoxycarbonyl, Ethoxycarbonyl, Methylamino, Ethylamino, n-Propylamino, Isopropylamino, n-Butylamino, sek.-Butylamino, iso-Butylamino, tert.-Butylamino, Phenyl, Naphthyl, Phenylamino, Naphthylamino, Phenylsulfonylamino oder Naphthylsulfonylamino, wobei jeder der sechs zuletzt genannten Reste im Arylteil einfach bis dreifach, gleichartig oder verschieden substituiert sein kann durch Fluor, Chlor, Brom, Cyano, Nitro, Formyl, Carboxy, Carbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methoxycarbonyl, Hydroximinomethyl, Hydroximinoethyl, Methoximinomethyl, Ethoximinomethyl, Methoximinoethyl oder Ethoximinoethyl und/oder durch jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy.
- R¹ und R²: stehen außerdem gemeinsam mit dem Stickstoffatom, an das sie gebunden sind, für ein cyclisches Imid der Formel 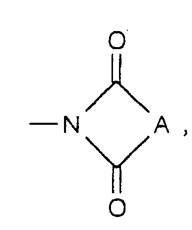 worin
A bevorzugt für Alkandiyl mit 2 oder 3 Kohlenstoffatomen oder Alkendiyl mit 2 oder 3 Kohlenstoffatomen steht, wobei die genannten Reste jeweils einfach bis dreifach, gleichartig oder verschieden substituiert sein können durch Fluor, Chlor, Brom, Cyano, Methyl, Ethyl, n- oder i-Propyl, n-, i-, s- oder t-Butyl, Methoxy, Ethoxy, n- oder i-Propoxy, Trifluormethyl, Trifluorethyl, Difluormethoxy, Trifluormethoxy, Difluorchlormethoxy und/oder Trifluorethoxy.
- X¹: steht bevorzugt für Wasserstoff, Fluor oder Chlor.
- X²: steht bevorzugt für Fluor oder Chlor.

Die oben allgemein aufgeführten oder in den Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Bevorzugte erfindungsgemäße Verbindungen sind auch Salze, die durch Umsetzung von Pyridyl-thiazolen der Formel 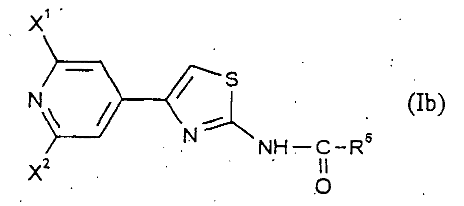 in welcher
R⁶, X¹ und X² die oben angegebenen Bedeutungen haben,
mit starken Basen der Formel

HOMe (IX)

in welcher
- Me: für ein Alkalimetallion, ein Äquivalent eines Erdalkalimetallions, ein Ammoniumion, ein Alkylammoniumion mit 1 bis 4 Kohlenstoffatomen, ein Dialkylammoniumion mit 1 bis 4 Kohlenstoffatomen in jeder Alkylgruppe oder ein Trialkylammoniumion mit 1 bis 4 Kohlenstoffatomen in jeder Alkylgruppe steht,
erhältlich sind.

Besonders bevorzugt sind derartige Salze von Pyridyl-thiazolen der Formel (Ib), in denen R⁶, X¹ und X² die oben als besonders bevorzugt genannten Bedeutungen haben und Me für ein Lithium-, Natrium- oder Kaliumion, für ein Äquivalent eines Magnesium-, Calcium-, Strontium- oder Bariumions, für ein Ammonium-, Methylammonium-, Ethylammoniumion-, Dimethylammonium-, Diethylammonium-, Trimethylammonium- oder Triethylammoniumion steht.

Bevorzugte erfindungsgemäße Stoffe sind auch Additionsprodukte aus Säuren und denjenigen Pyridyl-thiazolen der Formel (I), in denen X¹, X² und R die als bevorzugt angegebenen Bedeutungen haben.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure sowie Sulfonsäuren, wie z.B. p-Toluolsulfonsäure, 1,5-Naphthalindisulfonsäure oder Camphersulfonsäure, Saccharin und Thiosaccharin.

Verwendet man 4-Bromacetyl-2,6-dichlor-pyridin und Thioharnstoff als Ausgangsstoffe, so kann der Verlauf des erfindungsgemäßen Verfahrens (a) durch das folgende Formelschema veranschaulicht werden:

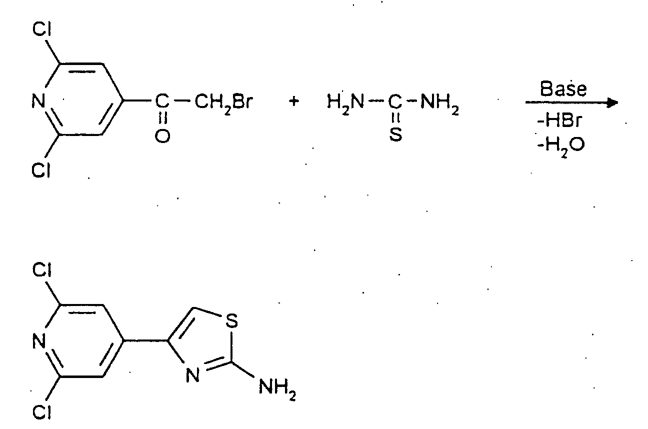

Verwendet man [4-(2,6-Dichlor-pyridin-4-yl)-thiazol-2-yl]-amin und Essigsäureanhydrid als Ausgangsstoffe, so kann der Verlauf des erfindungsgemäßen Verfahrens (b) nach der Variante (α) durch das folgende Formelschema veranschaulicht werden:

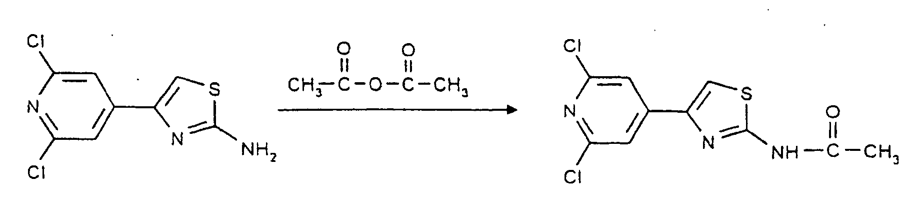

Verwendet man [4-(2,6-Dichlor-pyridin-4-yl)-thiazol-2-yl]-amin und 2-Trifluormethyl-phenyl-sulfonyl-isocyanat als Ausgangsstoffe, so kann der Verlauf des erfindungsgemäßen Verfahrens (b) nach der Variante (γ) durch das folgende Formelschema veranschaulicht werden:

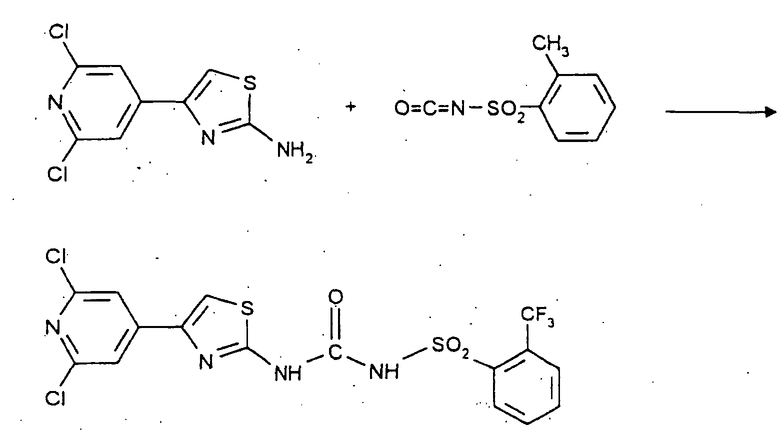

Verwendet man N-[4-(2,6-Dichlor-pyridin-4-yl)-thiazol-2-yl]-N'-(2-trifluormethylphenyl-sulfonyl)-harnstoff als Ausgangsstoff und Natriumhydroxid als Reaktionskomponente, so kann die Herstellung von Salzen nach dem erfindungsgemäßen Verfahren durch das folgende Formelschema veranschaulicht werden:

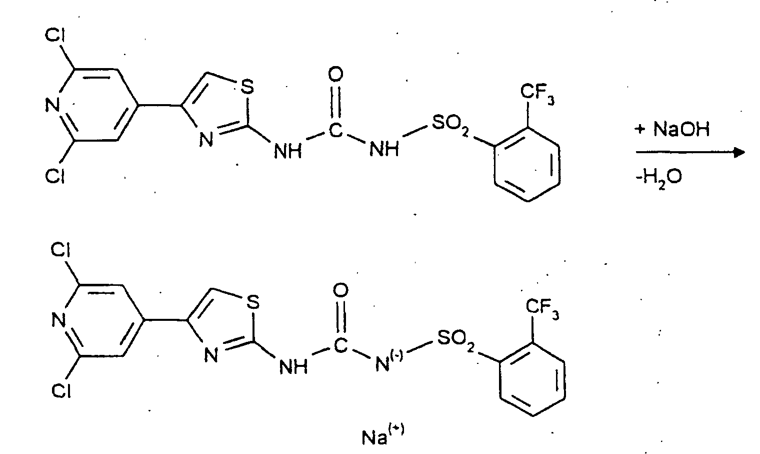

Das zuvor angegebene Salz kann auch in der tautomeren Form der Struktur 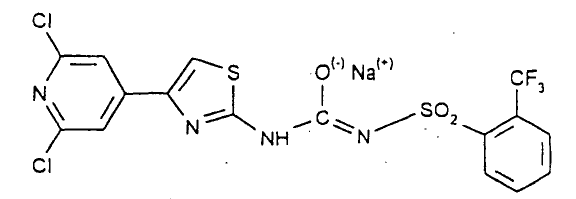 vorliegen.

Die zur Durchführung des erfindungsgemäßen Verfahrens (a) als Ausgangsstoffe benötigten Halogenacetylpyridine sind durch die Formel (II) allgemein definiert. In dieser Formel (II) haben X¹ und X² vorzugsweise diejenigen Bedeutungen, die bereits im Zusammenhang mit der Beschreibung der erfindungsgemäßen Verbindungen der Formel (I) als bevorzugt für X¹ und X² angegeben wurden. X³ steht bevorzugt für Chlor oder Brom.

Die Halogenacetyl-pyridine der Formel (II) sind teilweise bekannt (vgl. DE-A 1 811 833).

Die Halogenacetyl-pyridine der Formel 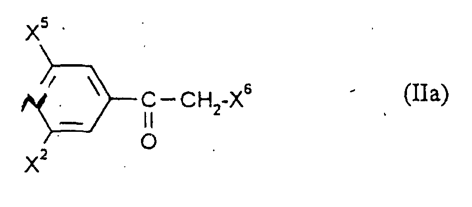 in welcher
- X²: für Halogen steht,
- X⁵: für Halogen steht und
- X⁶: für Chlor, Brom oder Iod steht,
sind neu.

Die Halogenacetyl-pyridine sind durch die Formel (IIa) allgemein definiert.
- X²: steht vorzugsweise für Fluor, Chlor oder Brom.
- X⁵: steht vorzugsweise für Fluor, Chlor oder Brom.
- X⁶: steht vorzugsweise für Chlor oder Brom.

Besonders bevorzugt sind Halogenacetyl-pyridine der Formel (IIa), in denen
- X²: für Fluor oder Chlor steht,
- X⁵: für Fluor oder Chlor steht und
- X⁶: für Chlor oder Brom steht.

Die Halogenacetyl-pyridine der Formel (IIa) lassen sich herstellen, indem man
c) Acetylpyridine der Formel 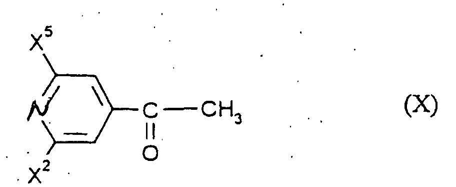 in welcher
   - X² und X⁵: die oben angegebenen Bedeutungen haben,
   mit Halogenierungsmitteln gegebenenfalls in Gegenwart eines Verdünnungsmittels und gegebenenfalls in Gegenwart eines Katalysators umsetzt.

In analoger Weise können die bekannten Halogenacetyl-pyridine der Formel (II) hergestellt werden.

Verwendet man 4-Acetyl-2,6-dichlor-pyridin als Ausgangsstoff und Brom als Halogenierungsmittel, so kann der Verlauf des Verfahrens (c) durch das folgende Formelschema veranschaulicht werden.

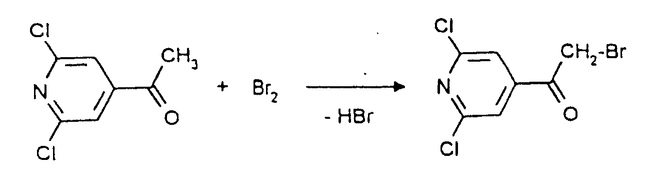

Die bei der Durchführung des Verfahrens (c) als Ausgangsstoffe benötigten Acetylpyridine sind durch die Formel (X) allgemein definiert. In dieser Formel haben X² und X⁵ vorzugsweise diejeinigen Bedeutungen, die bereits im

Zusammenhang mit der Beschreibung der Verbindungen der Formel (IIa) für diese Reste als bevorzugt genannt wurden.

Die Acetylpyridine der Formel (X) sind bisher noch nicht bekannt. Sie lassen sich herstellen, indem man
d) Malonester-Derivate der Formel 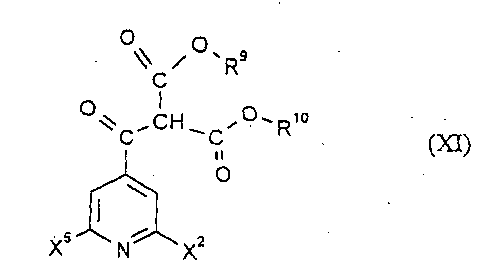 in welcher
   - X² und X⁵: die oben angegebenen Bedeutungen haben,
   - R⁹: für Alkyl steht und
   - R¹⁰: für Alkyl steht,
   mit Wasser gegebenenfalls in Gegenwart eines Verdünnungsmittels umsetzt.

Verwendet man 2,6-Dichlor-iso-nicotinoyl-malonsäure-dimethylester als Ausgangsstoff und Wasser als Reaktionskomponente, so kann der Verlauf des Verfahrens (d) durch das folgende Formelschema veranschaulicht werden.

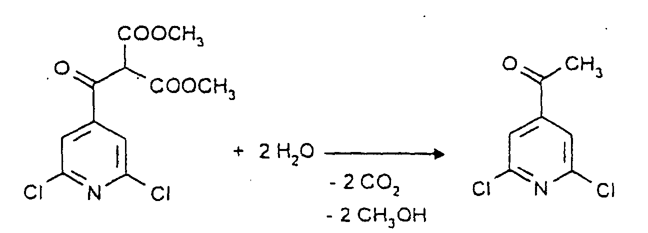

In einer speziellen Variante kann das Verfahren (d) auch so durchgeführt werden, daß nur eine partielle Verseifung und Decarboxylierung erfolgt. Die dabei entstehenden Ester der Formel 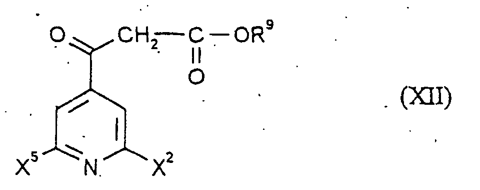 in welcher
X², X⁵ und R⁹ die oben. angegebenen Bedeutungen haben,
können isoliert werden und danach in einer weiteren Stufe unter den Reaktionsbedingungen des Verfahrens (d) zu Acetylpyridinen der Formel (X) umgesetzt werden.

Die bei der Durchführung des Verfahrens (d) als Ausgangsstoffe benötigten Malonester-Derivate sind durch die Formel (XI) allgemein definiert. In dieser Formel haben X² und X⁵ vorzugsweise diejenigen Bedeutungen, die bereits im Zusammenhang mit der Beschreibung der Verbindungen der Formel (IIa) für diese Reste als bevorzugt genannt wurden. R⁹ steht vorzugsweise für Alkyl mit 1 bis 6 Kohlenstoffatomen, besonders bevorzugt für Methyl, Ethyl, n-Propyl, Isopropyl, n-Butyl, iso-Butyl, sek.-Butyl oder tert.-Butyl. R¹⁰ steht ebenfalls vorzugsweise für Alkyl mit 1 bis 6 Kohlenstoffatomen, besonders bevorzugt für Methyl, Ethyl, n-Propyl, Isopropyl, n-Butyl, iso-Butyl, sek.-Butyl oder tert.-Butyl.

Die Malonester-Derivate der Formel (XI) sind bisher noch nicht bekannt. Sie lassen sich herstellen, indem man
e) Isonicotinsäure-halogenide der Formel 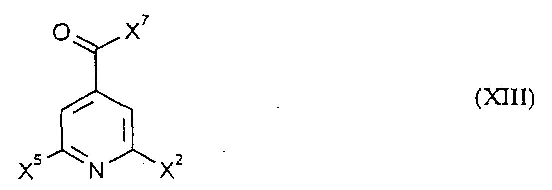
in welcher
- X² und X⁵: die oben angegebenen Bedeutungen haben und
- X⁷: für Halogen steht,
mit Malonestern der Formel 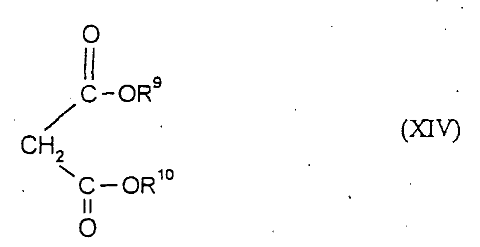 in welcher
- R⁹ und R¹⁰: die oben angegebenen Bedeutungen haben,
gegegebenenfalls in Gegenwart eines Verdünnungsmittels, sowie gegebenenfalls in Gegenwart eines Säurebindemittels, und gegebenenfalls in Gegenwart eines Katalysators umsetzt.

Die Malonester-Derivate der Formel (XI) sowie deren Salze und Säure-Addukte sind sehr gut zum Schutz von Pflanzen gegen Befall durch unerwünschte Mikroorganismen geeignet.

Verwendet man 2,6-Dichlor-isonicotinsäure-chlorid als Ausgangsstoff und Malonsäure-dimethylester als Reaktionskomponente, so kann der Verlauf des Verfahrens (e) durch das folgende Formelschema veranschaulicht werden.

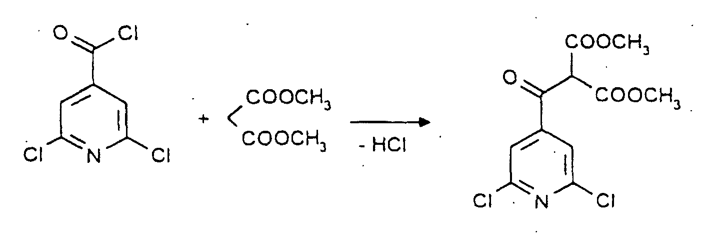

Die Malonester-Derivate der Formel (XI) liegen im allgemeinen in Form von Tautomeren-Gemischen vor, die durch die folgenden Formeln charakterisiert werden können:

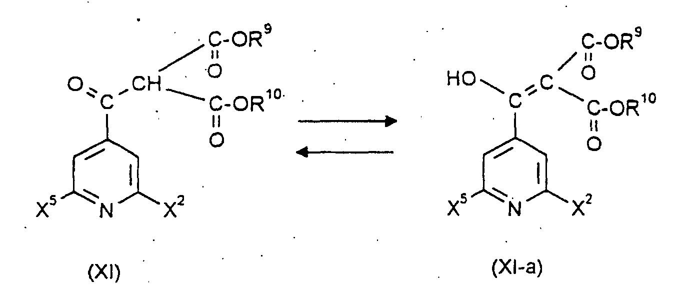

Die bei der Durchführung des Verfahrens (e) als Ausgangsstoffe benötigten Isonicotinsäure-halogenide sind durch die Formel (XIII) allgemein definiert. In dieser Formel haben X² und X⁵ vorzugsweise diejenigen Bedeutungen, die bereits im Zusammenhang mit der Beschreibung der Verbindungen der Formel (IIa) für diese Reste als bevorzugt genannt wurden. X⁷ steht vorzugsweise für Chlor.

Die Isonicotinsäure-halogenide der Formel (XIII) sind bekannt oder lassen sich nach prinzipiell bekannten Verfahren herstellen (vgl. J. Chem. Soc. 71 (1897), 1076).

Die bei der Durchführung des Verfahrens (e) als Reaktionskomponenten benötigten Malonester sind durch die Formel (XIV) allgemein definiert. In dieser Formel haben R⁹ und R¹⁰ vorzugsweise diejenigen Bedeutungen, die bereits im Zusammenhang mit der Beschreibung der Malonester-Derivate der Formel (XI) für diese Reste als bevorzugt genannt wurden.

Die Malonester der Formel (XIV) sind bekannt oder lassen sich nach bekannten Methoden herstellen.

Als Verdünnungsmittel können bei der Durchführung des Verfahrens (e) alle üblichen inerten organischen Solventien eingesetzt werden. Vorzugsweise verwendbar sind polare, aprotische Lösungsmittel, wie Ether, wie Dioxan, Tetrahydrofuran oder 1,2-Dimethoxyethan, ferner Amide, wie N,N-Dimethylformamid, N,N-Dimethylacetamid oder N-Methylpyrrolidon, weiterhin Nitrile, wie Acetonitril, Propionitril, n-Butyronitril oder iso-Butyronitril, außerdem Sulfoxide, wie Dimethylsulfoxid, und auch Sulfone, wie Sulfolan.

Als Säurebindemittel können bei der Durchführung des Verfahrens (e) alle für derartige Reaktionen üblichen Säureakzeptoren eingesetzt werden. Vorzugsweise verwendbar sind tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Katalysatoren kommen bei der Durchführung des Verfahrens (e) alle für derartige Reaktionen üblichen Reaktionsbeschleuniger in Frage. Vorzugsweise verwendbar sind Salze von mehrwertigen Metallen, wie Magnesiumchlorid, Zinkchlorid, Kupfer-(II)-sulfat oder Eisen-(III)-chlorid.

Die Reaktionstemperaturen können bei der Durchführung des Verfahrens (e) innerhalb eines bestimmten Bereiches variiert werden. Im allgemeinen arbeitet man bei Temperaturen zwischen -20°C und +40°C, vorzugsweise zwischen -10°C und +30°C.

Sowohl bei der Durchführung des Verfahrens (e) als auch der Verfahren (c) und (d) arbeitet man im allgemeinen unter Atmosphärendruck. Es ist jedoch auch möglich, jeweils unter erhöhtem oder vermindertem Druck zu arbeiten.

Bei der Durchführung des Verfahrens (e) setzt man auf 1 Mol an Isonicotinsäurehalogenid der Formel (XII) im allgemeinen 0,5 bis 2 Mol, vorzugsweise 0,8 bis 1,5 Mol an Malonester der Formel (XIV) ein. Die Aufarbeitung erfolgt nach üblichen Methoden. Im allgemeinen verfährt man in der Weise, daß man ansäuert, das dabei erhaltene Gemisch mit einem mit Wasser wenig mischbaren organischen Lösungsmittel extrahiert, die vereinigten organischen Phasen wäscht, gegebenenfalls trocknet und einengt. Das verbleibende Produkt kann gegebenenfalls nach üblichen Methoden von eventuell noch vorhandenen Verunreinigungen befreit werden.

Als Verdünnungsmittel kommen bei der Durchführung des Verfahrens (d) vorzugsweise mit Wasser mischbare, organische Solventien in Betracht. Als Beispiele seien Tetrahydrofuran, Acetonitril, Dimethylsulfoxid und Sulfolan genannt.

Die Reaktionstemperaturen können bei der Durchführung des Verfahrens (d) innerhalb eines größeren Bereiches variiert werden. Im allgemeinen arbeitet man bei Temperaturen zwischen 50°C und 200°C, vorzugsweise zwischen 80°C und 180°C.

Bei der Durchführung des Verfahrens (d) setzt man auf 1 Mol an Malonester-Derivat der Formel (XI) im allgemeinen 2 bis 5 Mol, vorzugsweise 2 bis 2,5 Mol Wasser ein. Die Aufarbeitung erfolgt nach üblichen Methoden. Im allgemeinen verfährt man in der Weise, daß man mit Eiswasser versetzt, das entstehende Gemisch mehrfach mit einem mit Wasser wenig mischbaren organischen Lösungsmittel extrahiert, die vereinigten organischen Phasen mit Wasser in Gegenwart einer Base wäscht, dann trocknet, einengt und den verbleibenden Rückstand destilliert.

Als Halogenierungsmittel kommen bei der Durchführung des Verfahrens (c) vorzugsweise Brom, Chlor, N-Brom-succinimid oder N-Jod-succinimid in Betracht.

Als Verdünnungsmittel können bei der Durchführung des Verfahrens (c) alle üblichen inerten, organischen Solventien eingesetzt werden. Vorzugsweise verwendbar sind gegebenenfalls halogenierte aliphatische, alicyclische oder aromatische Kohlenwasserstoffe, wie Hexan, Cyclohexan, Benzol, Dichlormethan, Chloroform, Tetrachlormethan oder Trichlorethan.

Als Katalysatoren kommen bei der Durchführung des Verfahrens (c) vorzugsweise Lewis-Säuren, wie Aluminium-trichlorid in Betracht.

Die Reaktionstemperaturen können auch bei der Durchführung des Verfahrens (c) innerhalb eines größeren Bereiches variiert werden. Im allgemeinen arbeitet man bei Temperaturen zwischen -20°C und +120°C, vorzugsweise zwischen 10°C und 50°C.

Bei der Durchführung des Verfahrens (c) setzt man auf 1 Mol an Acetylpyridin der Formel (X) im allgemeinen 0,5 bis 5 Mol, vorzugweise 0,8 bis 1,2 Mol an Halogenierungsmittel ein. Die Aufarbeitung erfolgt nach üblichen Methoden. Im allgemeinen verfährt man in der Weise, daß man das Reaktionsgemisch mit Wasser versetzt, die organische Phase nacheinander mit Wasser in Gegenwart von Base und dann mit Wasser wäscht, anschließend trocknet und einengt. Das anfallende Produkt kann nach üblichen Methoden von eventuell noch enthaltenen Verunreinigungen befreit werden.

Die zur Durchführung des erfindungsgemäßen Verfahrens (a) weiterhin als Ausgangsstoffe benötigten Thioverbindungen sind durch die Formel (III) allgemein definiert. In dieser Formel (III) hat R vorzugsweise diejenigen Bedeutungen, die bereits im Zusammenhang mit der Beschreibung der erfindungsgemäßen Verbindungen der Formel (I) als bevorzugt für R angegeben wurden.

Die Thioverbindungen der Formel (III) sind bekannte Synthesechemikalien.

Die zur Durchführung des erfindungsgemäßen Verfahrens (b) als Ausgangsstoffe benötigten Aminothiazole der Formel (Ia) sind erfindungsgemäße Stoffe, die sich nach dem erfindungsgemäßen Verfahren (a) herstellen lassen.

Die bei der Durchführung des erfindungsgemäßen Verfahrens (b, Variante α) als Reaktionskomponenten benötigten Carbonsäureanhydride sind durch die Formeln (IV) und (V) allgemein definiert.

In diesen Formeln haben R⁵ und A vorzugsweise diejenigen Bedeutungen, die bereits im Zusammenhang mit der Beschreibung der erfindungsgemäßen Pyridyl-thiazole der Formel (I) für diese Reste als bevorzugt genannt wurden.

Die Carbonsäureanhydride der Formeln (IV) und (V) sind bekannt oder lassen sich nach bekannten Verfahren herstellen.

Die bei der Durchführung des erfindungsgemäßen Verfahrens (b, Variante β) als Reaktionskomponenten benötigten Carbonsäurehalogenide sind durch die Formel (VI) allgemein definiert. In dieser Formel hat R⁵ vorzugsweise diejenigen Bedeutungen, die bereits im Zusammenhang mit der Beschreibung der erfindungsgemäßen Pyridyl-thiazole für diesen Rest als bevorzugt genannt wurden. X⁴ steht vorzugsweise für Chlor oder Brom.

Die Carbonsäurehalogenide der Formel (VI) sind bekannt oder lassen sich nach bekannten Verfahren herstellen.

Die bei der Durchführung des erfindungsgemäßen Verfahrens (b, Variante γ) als Reaktionskomponenten benötigten Isocyanate sind durch die Formel (VII) allgemein definiert. In dieser Formel steht R⁷ bevorzugt für Methyl, Ethyl, n-Propyl, Isopropyl, n-Butyl, sek.-Butyl, iso-Butyl, tert.-Butyl, Phenyl, Naphthyl, Phenylsulfonyl oder Naphthylsulfonyl, wobei jeder der vier zuletzt genannten Reste im Arylteil einfach bis dreifach, gleichartig oder verschieden substituiert sein kann durch Fluor, Chlor, Brom, Cyano, Nitro, Formyl, Carboxy, Carbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methoxycarbonyl, Hydroximinomethyl, Hydroximinoethyl, Methoximinomethyl, Ethoximinomethyl, Methoximinoethyl oder Ethoximinoethyl und/oder durch jeweils gegebenenfalls einfach bis vierfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Methylendioxy oder Ethylendioxy.

Die Isocyanate der Formel (VII) sind bekannt oder lassen sich nach bekannten Verfahren herstellen.

Die bei der Durchführung des erfindungsgemäßen Verfahrens (b, Variante δ) als Reaktionskomponenten benötigten Acetale sind durch die Formel (VIII) allgemein definiert. In dieser Formel haben R³ und R⁴ vorzugsweise diejenigen Bedeutungen, die bereits im Zusammenhang mit der Beschreibung der erfindungsgemäßen Pyridyl-thiazole der Formel (I) für diese Reste als bevorzugt genannt wurden. R⁸ steht für Methyl oder Ethyl.

Als Verdünnungsmittel kommen bei der Durchführung des erfindungsgemäßen Verfahrens (a) alle üblichen, inerten organischen Solventien in Betracht. Vorzugsweise verwendbar sind aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid; Sufone, wie Sulfolan.

Als Säurebindemittel kommen bei der Durchführung des erfindungsgemäßen Verfahrens (a) alle üblichen anorganischen oder organischen Basen in Frage. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natriummethylat, Natrium-ethylat, Kaliumtert.-butylat, Natriumhydroxid, Kaliumhydroxid, Natriumacetat, Kaliumacetat, Calciumacetat, Natriumcarbonat, Kaliumcarbonat, Kaliumhydrogencarbonat oder Natriumhydrogencarbonat, ferner Ammoniumverbindungen, wie Ammoniumhydroxid, Ammoniumacetat und Ammoniumcarbonat, und außerdem tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Es ist aber auch möglich, bei der Durchführung des erfindungsgemäßen Verfahrens (a) in Abwesenheit von zusätzlichen Säurebindemitteln zu arbeiten.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen -20°C und +100°C, vorzugsweise zwischen 0°C und 80°C.

Sowohl bei der Durchführung des erfindungsgemäßen Verfahrens (a) als auch des Verfahrens (b) arbeitet man im allgemeinen unter Normaldruck. Es ist jedoch jeweils auch möglich, unter erhöhtem oder vermindertem Druck, z.B. zwischen 0,1 bar und 10 bar, zu arbeiten.

Bei der Durchführung des erfindungsgemäßen Verfahrens (a) setzt man auf 1 Mol an Halogenacetyl-pyridin der Formel (II) im allgemeinen 0,8 bis 10 Mol, vorzugsweise 1 bis 5 Mol an Thioverbindung der Formel (III) ein. Die Aufarbeitung erfolgt nach üblichen Methoden.

Als Verdünnungsmittel kommen bei der Durchführung des erfindungsgemäßen Verfahrens (b) alle üblichen, inerten organischen Solventien in Betracht. Vorzugsweise verwendbar sind im Falle der Varianten (α), (β) und (γ) alle diejenigen Lösungsmittel, die bereits im Falle des erfindungsgemäßen Verfahrens (a) als bevorzugt genannt wurden. Bei der Durchführung der Variante (δ) können auch Alkohole, wie Methanol oder Ethanol, als Verdünnungsmittel eingesetzt werden.

Als Säurebindemittel kommen bei der Durchführung des erfindungsgemäßen Verfahrens (b), Varianten α und β, alle für derartige Umsetzungen üblichen Säureakzeptoren in Betracht. Vorzugsweise verwendbar sind dabei diejenigen Säurebindemittel, die bereits im Falle des erfindungsgemäßen Verfahrens (a) als bevorzugt genannt wurden.

Die gesonderte Zugabe eines Katalysators erübrigt sich im allgemeinen bei der Durchführung der Varianten (α) und (β) des erfindungsgemäßen Verfahrens (b).

Bei der Durchführung der Varianten (γ) und (δ) des erfindungsgemäßen Verfahrens (b) ist die Zugabe eines Säurebindemittels im allgemeinen nicht erforderlich.

Als Katalysatoren kommen bei der Durchführung der Variante (γ) des erfindungsgemäßen Verfahrens (b) alle für derartige Umsetzungen üblichen Reaktionsbeschleuniger in Frage. Vorzugsweise verwendbar sind Amine, wie Pyridin, Dimethylaminopyridin und Diazabicyclo-undecen (DBU).

Als Katalysatoren kommen bei der Durchführung der Variante (δ) des erfindungsgemäßen Verfahrens (b) alle für derartige Umsetzungen üblichen Reaktionsbeschleuniger in Betracht. Vorzugsweise verwendbar sind Säuren, wie Schwefelsäure, Chlorwasserstoffsäure und Toluolsulfonsäure, und außerdem auch saure Ionenaustauscher.

Auch bei der Durchführung des erfindungsgemäßen Verfahrens (b) können die Reaktionstemperaturen innerhalb eines größeren Bereiches variiert werden. Im allgemeinen arbeitet man bei Temperaturen zwischen 0°C und 150°C, vorzugsweise zwischen 10°C und 120°C.

Bei der Durchführung des erfindungsgemäßen Verfahrens (b) setzt man auf 1 Mol an Aminothiazol der Formel (Ia) im allgemeinen 0,8 bis 15 Mol, vorzugsweise 1 bis 8 Mol an Reaktionskomponente der Formel (IV), (V), (VI), (VII) oder (VIII) ein. Die Aufarbeitung erfolgt nach üblichen Methoden.

Bei der Herstellung von Salzen erfindungsgemäßer Verbindungen verfährt man im allgemeinen in der Weise, daß man ein Pyridyl-thiazol der Formel (Ib) in einem inerten organischen Solvens, wie z.B. Methanol oder Ethanol, löst und bei Raumtemperatur oder auch bei etwas erhöhter Temperatur mit einer starken Base der Formel (IX) versetzt. Die Isolierung und gegebenenfalls erforderliche Reinigung der dabei entstehenden Salze erfolgt nach üblichen Methoden.

Die erfindungsgemäßen Pyridyl-thiazole der Formel (I) können auch in Säureadditions-Salze überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe weisen eine starke resistenz-induzierende Wirkung in Pflanzen auf. Sie eignen sich daher zur Erzeugung von Resistenz in Pflanzen gegen Befall durch unerwünschte Mikroorganismen.

Unter resistenzinduzierenden Stoffen sind im vorliegenden Zusammenhang solche Substanzen zu verstehen, die in der Lage sind, das Abwehrsystem von Pflanzen so zu stimulieren, daß die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um in Pflanzen innerhalb eines gewissen Zeitraumes nach der Behandlung Resistenz gegen den Befall durch die genannten Schaderreger zu erzeugen. Der Zeitraum, innerhalb dessen Resistenz herbeigeführt wird, erstreckt sich im allgemeinen von 1 bis 10 Tage, vorzugsweise 1 bis 7 Tage nach der Behandlung der Pflanzen mit den Wirkstoffen.

Die erfindungsgemäßen Wirkstoffe weisen neben der resistenzinduzierenden Wirkung auch eine starke mikrobizide Wirkung auf und werden auch zur direkten Bekämpfung von unerwünschten Mikroorganismen praktisch eingesetzt. Die Wirkstoffe sind für den Gebrauch als Pflanzenschutzmittel, insbesondere als Fungizide geeignet.

Zu den unerwünschten Mikroorganismen im Pflanzenschutz gehören Pilze aus den Klassen Plasmodiophoromycetes, Oomycetes, Chytridiomycetes, Zygomycetes, Ascomycetes, Basidiomycetes, Deuteromycetes.

Beispielhaft seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen, erlaubt eine Behandlung von oberirdischen Pflanzenteilen, sowie auch eine Behandlung von Pflanz- und Saatgut und des Bodens.

Dabei lassen sich die erfindungsgemäßen Wirkstoffe mit besonders gutem Erfolg zur Bekämpfung von Getreidekrankheiten, wie beispielsweise gegen Erysiphe-Arten, oder von Krankheiten im Wein-, Obst- und Gemüseanbau, wie beispielsweise gegen Plasmopara- oder Venturia-Arten, oder von Reiskrankheiten, wie beispielsweise gegen Pyricularia-Arten einsetzen. Mit gutem Erfolg lassen sich mit den erfindungsgemäßen Wirkstoffen auch weitere Pflanzenkrankheiten, wie beispielsweise Septoria-, Cochliobolus-, Pyrenophora-, und Pseudocercosporella-Arten bekämpfen.

Die Wirkstoffe können in Abhängigkeit von ihren jeweiligen physikalischen und/oder chemischen Eigenschaften in übliche Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie z.B. Gummiarabicum, Polyvinylalkohol, Polyvinylacetat, sowie natürliche Phospholipide, wie Kephaline und Lecithine und synthetische Phospholipide. Weitere mögliche Additive sind mineralische und vegetabile Öle.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

In vielen Fällen werden dabei synergistische Wirkungen beobachtet.

Für die Mischungen kommen beispielsweise infrage:

### Fungizide:

2-Aminobutan, 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoximino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb,
Cymoxanil, Cyproconazole, Cyprofuram,
Dichlorophen, Diclobutrazol, Diclofluanid, Diclomezin, Dicloran, Diethofencarb,
Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropi-morph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol,
Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole,. Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin,
Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb,
Fenoxycarb, Fenpropathrin, Fenpyrad, Fenpyroximat; Fenthion, Fenvalerate,
Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lambda-cyhalothrin, Lufenuron,
Malathion, Mecarbam, Mevinphos, Mesulfenphos, Metaldehyd, Methacrifos,
Methamidophos, Methidathion, Methiocarb, Methomyl, Metolcarb, Milbemectin,
Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet,
Phosphamidon, Phoxim, Pirimicarb, Pirimiphos M, Pirimiphos A,
Profenophos, Promecarb, Propaphos, Propoxur, Prothiofos, Prothiophos, Prothoat,
Pymetrozin, Pyrachlophos, Pyridaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Die erfindungsgemäßen Wirkstoffe können auch mit anderen bekannten Wirkstoffen, wie Herbiziden oder auch mit Düngemitteln und Wachstumsregulatoren vermischt werden.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die Herstellung und die Verwendung von erfindungsgemäßen Wirkstoffen werden durch die folgenden Beispiele veranschaulicht.

### Herstellungsbeispiele

### Beispiel 1

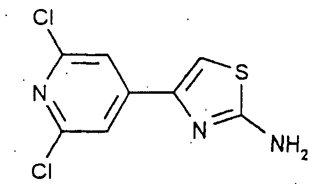

931,5 g (3,46 mol) 4-Bromacetyl-2,6-dichlorpyridin und 318,2 g (4,18 mol) Thioharnstoff werden in 4,2 Litern Aceton unter Rühren zusammengegeben, wobei die Temperatur auf 45°C steigt. Die Mischung wird weitere 3 Stunden gerührt. Der dabei anfallende Feststoff wird abgesaugt, mit wenig Aceton gewaschen und unter weiterem Absaugen getrocknet. Man suspendiert das so erhaltene Produkt bei Raumtemperatur in 2 Litern Wasser, stellt durch Zugabe von konzentrierter, wäßriger Natronlauge auf einen pH-Wert von 10 ein, saugt wiederum ab, wäscht mit 3 Litern Wasser nach und trocknet bei 50°C unter vermindertem Druck. Das so erhaltene Rohprodukt wird mit 30 Litern tert.-Amyl-methyl-ether verrührt, und das dabei anfallende Gemisch wird abgesaugt. Das Filtrat wird unter vermindertem Druck eingeengt und der verbleibende Rückstand wird bei 50°C unter vermindertem Druck getrocknet. Der nach dem Absaugen des tert.-Amyl-methylether-Gemisches isolierte Feststoff wird in 1 Liter heißem Ethanol gelöst. Die entstehende Lösung wird filtriert und dann auf 5°C abgekühlt. Der dabei abgeschiedene Feststoff wird ebenfalls abgesaugt und bei 50°C unter vermindertem Druck getrocknet. Man erhält auf diese Weise insgesamt 600,1 g (70,5 % der Theorie) an [4-(2,6-Dichlorpyridin-4-yl)-thiazol-2-yl]-amin als Festsubstanz vom Schmelzpunkt 239 bis 247°C.

### Herstellung von Ausgangsstoffen:

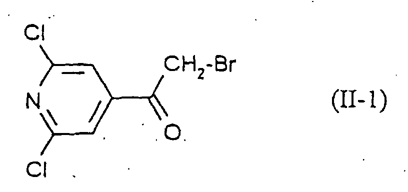

Zu einer Lösung von 525 g (2,763 Mol) 4-Acetyl-2,6-dichlor-pyridin in 1,37 Litern Dichlormethan gibt man zunächst 2 g Aluminiumchlorid und tropft dann 417,6 g (2,613 Mol) Brom unter Rühren bei 30 bis 35°C innerhalb von 30 Minuten zu. Nach beendeter Zugabe wird das Reaktionsgemisch noch eine Stunde bei 35°C nachgerührt und dann mit 1,2 l Wasser versetzt. Die organische Phase wird abgetrennt und nacheinander zunächst mit 1,2 l gesättigter, wäßriger Natriumhydrogencarbonat-Lösung und dann zweimal mit je 1,2 l Wasser gewaschen. Man trocknet über Natriumsulfat und engt dann unter vermindertem Druck ein. Der verbleibende Rückstand wird mit 2 l Petrolether verrührt, dann abgesaugt und portionsweise mit insgesamt 1 Liter Petrolether nachgewaschen. Das anfallende Produkt wird bei 50°C unter vermindertem Druck getrocknet. Man erhält auf diese Weise 592,2 g (79,7 % der Theorie) an 4-Brom-acetyl-2,6-dichlorpyridin in Form einer Festsubstanz.
¹H-NMR-Spektrum (CDCl₃/TMS): δ = 4,42 (s, 2H); 7,74 (s, 2H) ppm

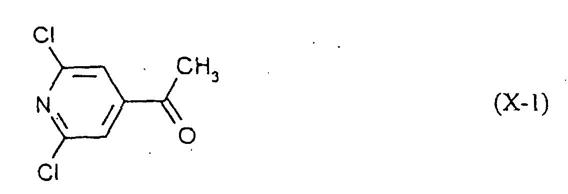

1328 g (4,34 Mol) 2,6-Dichlor-isonicotinoyl-malonsäure-dimethylester werden in einem Gemisch aus 3,45 l Dimethylsulfoxid und 156,2 g (8,68 Mol) Wasser gelöst. Das Reaktionsgemisch wird auf 140°C erhitzt und 30 Minuten bei dieser Temperatur gerührt. Danach wird das Reaktionsgemisch auf 10°C abgekühlt und dann auf 8,4 l Eiswasser gegeben. Man extrahiert das Gemisch zweimal mit insgesamt 3,6 l Dichlormethan und wäscht die vereinigten organischen Phasen einmal mit 1,5 l gesättigter, wäßriger Natriumhydrogencarbonat-Lösung. Nach dem Trocknen über Natriumsulfat wird die organische Phase unter vermindertem Druck eingeengt. Der verbleibende Rückstand wird unter vermindertem Druck destilliert.

Man erhält auf diese Weise 506,0 g (60 % der Theorie) an 4-Acetyl-2,6-dichlorpyridin vom Siedepunkt 98°C bei 0,5 mbar.

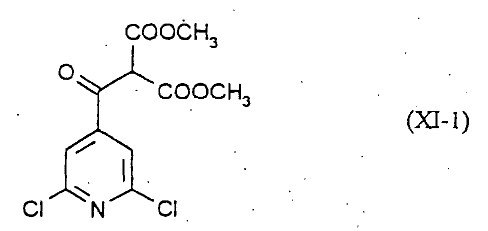

2 312 g (24,28 Mol) Magnesiumchlorid werden unter Kühlung in 18 l Acetonitril eingetragen. Nach dem Abklingen der exothermen Reaktion wird die Lösung auf 0°C gekühlt und unter Rühren innerhalb von 45 Minuten tropfenweise mit 3210 g (24,3 Mol) Malonsäuredimethylester versetzt. Danach werden bei 0°C unter Rühren innerhalb von 1,5 Stunden 4920 g (48,62 Mol) Triethylamin zugetropft. Nach beendeter Zugabe wird weitere 15 Minuten bei 0°C gerührt. Anschließend wird bei 0°C unter Rühren innerhalb von 20 Stunden eine Lösung von 4742 g (22,53 Mol) 2,6-Dichlor-isonicotinsäure-chlorid in 6670 ml Acetonitril zugetropft. Es wird zunächst eine Stunde bei 0°C und dann 16 Stunden.. unter langsamem Erwärmen auf Raumtemperatur nachgerührt. Anschließend wird das Reaktionsgemisch unter Kühlung auf 0 bis 10°C unter Rühren tropfenweise mit 15,9 l konzentrierter Salzsäure versetzt. Danach versetzt man unter Rühren mit 15,9 l Dichlormethan und 6,4 l Wasser und trennt dann die organische Phase ab. Die wäßrige Phase wird noch einmal mit 7,9 l Dichlormethan extrahiert. Die vereinigten organischen Phasen werden zweimal mit je 4 l Wasser gewaschen und dann unter vermindertem Druck eingeengt. Der verbleibende Rückstand wird mit 9,5 l Wasser verrührt, dann abgesaugt und nacheinander zuerst mit 6,3 l Wasser und dann zweimal mit je 7,9 l Petrolether nächgewaschen. Das anfallende Produkt wird bei 40°C unter vermindertem Druck getrocknet. Man erhält auf diese Weise 6,17 kg (89,2 % der Theorie) an 2,6-Dichlor-isonicotinoyl-malonsäure-dimethylester.
¹H-NMR-Spektrum (CDCl₃/TMS): δ = 3,68; 3,84; 3,92 und 7,39 ppm.

### Beispiel 2

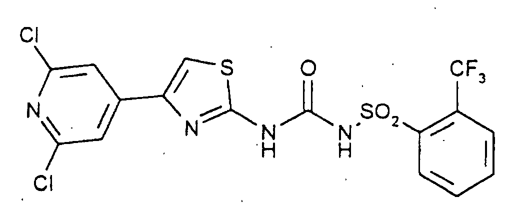

Zu einer Lösung von 7,38 g (0,03 mol) [4-(2,6-Dichlorpyridin-4-yl)-thiazol-2-yl]-amin in 180 ml Acetonitril werden bei 75°C unter Rühren 7,53 g (0,03 mol) 2-Trifluormethylphenylsulfonylisocyanat innerhalb von 5 Minuten zugegeben. Man rührt das Reaktionsgemisch weitere 3 Stunden bei 75°C, kühlt dann auf Raumtemperatur ab, saugt den ausgefallenen Feststoff ab, wäscht zweimal mit jeweils 10 ml Acetonitril und trocknet bei 45°C unter vermindertem Druck. Man erhält auf diese Weise 14 g (93,9 % der Theorie) an N-[4-(2,6-Dichlor-pyridin-4-yl)-thiazol-2-yl]-N'-(2-trifluormethyl-phenylsulfonyl)-harnstoff in Form einer Festsubstanz mit einem Schmelzpunkt von >280°C.

### Beispiel 3

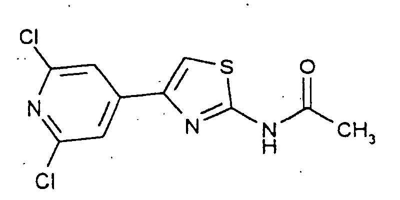

7,38 g (0,03 mol) [4-(2,6-Dichlorpyridin-4-yl)-thiazol-2-yl]-amin und 70 ml Essigsäureanhydrid werden 30 Minuten unter Rückfluß erhitzt, wobei sich zunächst eine Lösung bildet, dann aber ein Niederschlag ausfällt. Man läßt das Reaktionsgemisch 16 Stunden bei Raumtemperatur stehen, fügt dann 200 ml Eiswasser hinzu und saugt ab. Der Rückstand wird zunächst mehrmals mit Wasser gewaschen, dann mit 200 ml 5 %iger, wäßriger Natriumhydrogencarbonat-Lösung digeriert und erneut abgesaugt. Das Produkt wird bei 50°C unter vermindertem Druck getrocknet. Man erhält auf diese Weise 8,1 g (93,8 % der Theorie) an N-[4-(2,6-Dichlor-pyridin-4-yl)-thiazol-2-yl]-acetamid in Form einer Festsubstanz vom Schmelzpunkt 301°C.

### Beispiel 4

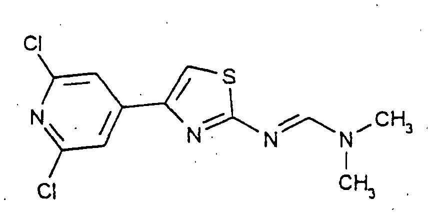

Zu einer Mischung von 7,38 g (0,03 mol) [4-(2,6-Dichlorpyridin-4-yl)-thiazol-2-yl]-amin und 50 ml Methanol gibt man bei 20 °C unter Rühren 3,93 g (0,033 mol) Dimethylformamiddimethylacetal und rührt noch 2 Stunden bei 50°C. Danach wird das Reaktionsgemisch auf 10°C abgekühlt und abgesaugt. Der Rückstand wird mit 10 ml kaltem Methanol gewaschen und anschließend bei 45°C unter vermindertem Druck getrocknet. Man erhält auf diese Weise 7,5 g (83 % der Theorie) an N-[4-(2,6-Dichlor-pyridin-4-yl)-thiazol-2-yl]-N,N'-dimethylformamidin in Form einer Festsubstanz vom Schmelzpunkt 145°C.

Nach den zuvor angegebenen Methoden werden auch die in der folgenden Tabelle aufgeführten Verbindungen der Formel 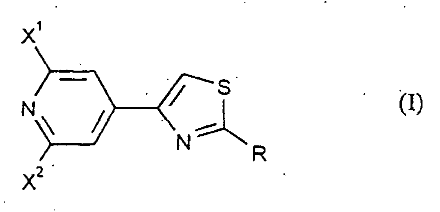 hergestellt.

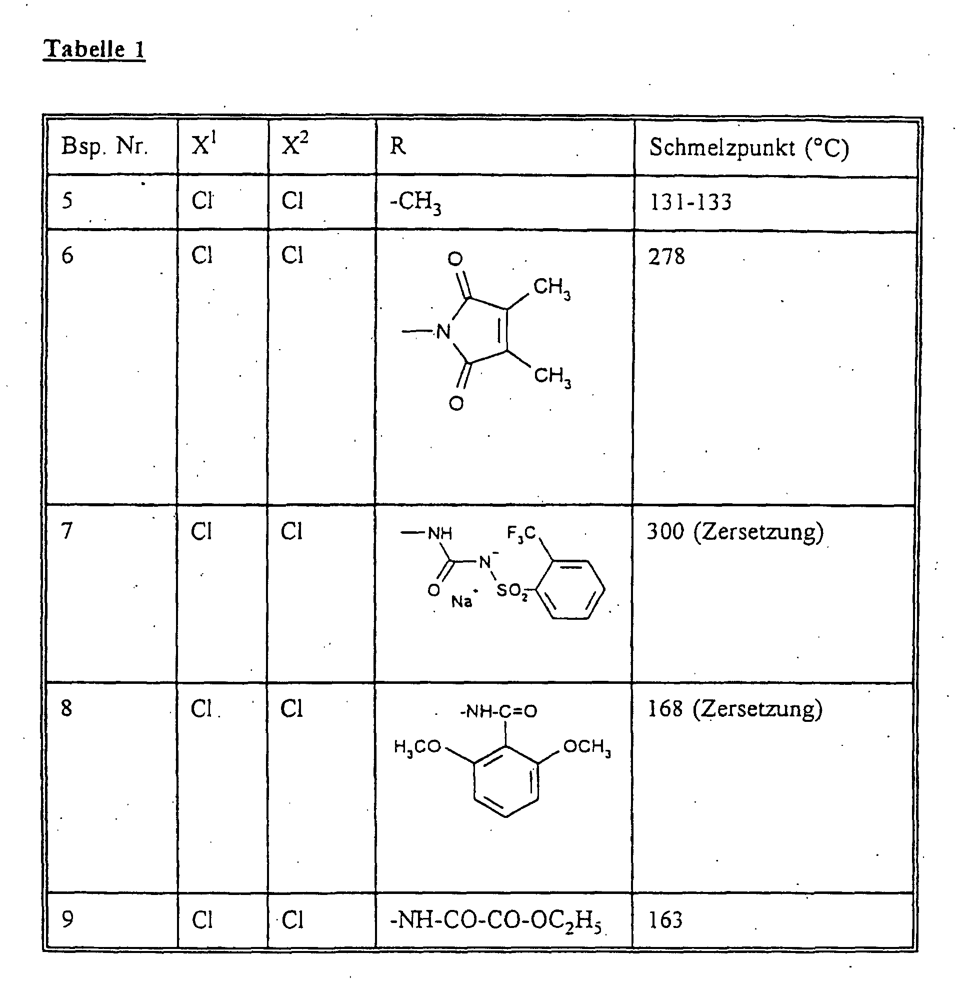

### Verwendungsbeispiele

### Beispiel A

### Plasmopara-Test (Reben) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100% relativer Luftfeuchtigkeit. Anschließend werden die Pflanzen 5 Tage im Gewächshaus bei 21°C und ca. 90% Luftfeuchtigkeit aufgestellt. Die Pflanzen werden dann angefeuchtet und 1 Tag in eine Feuchtkammer gestellt.

6 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

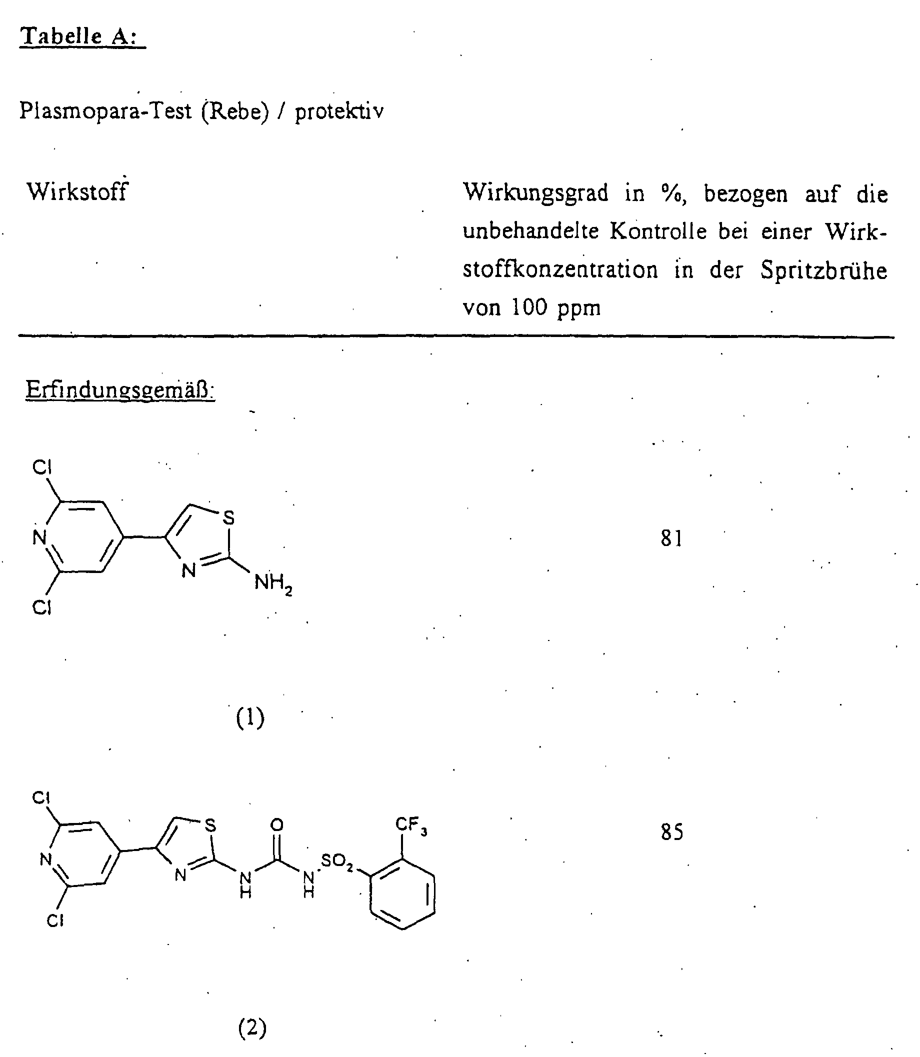

### Beispiel B

### Venturia-Test (Apfel) / protektiv

| | |
|---|---|
| Lösungsmittel | 4,7 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkönzentrationen und Versuchsergebnisse gehen aus der folgenden Tabelle hervor.

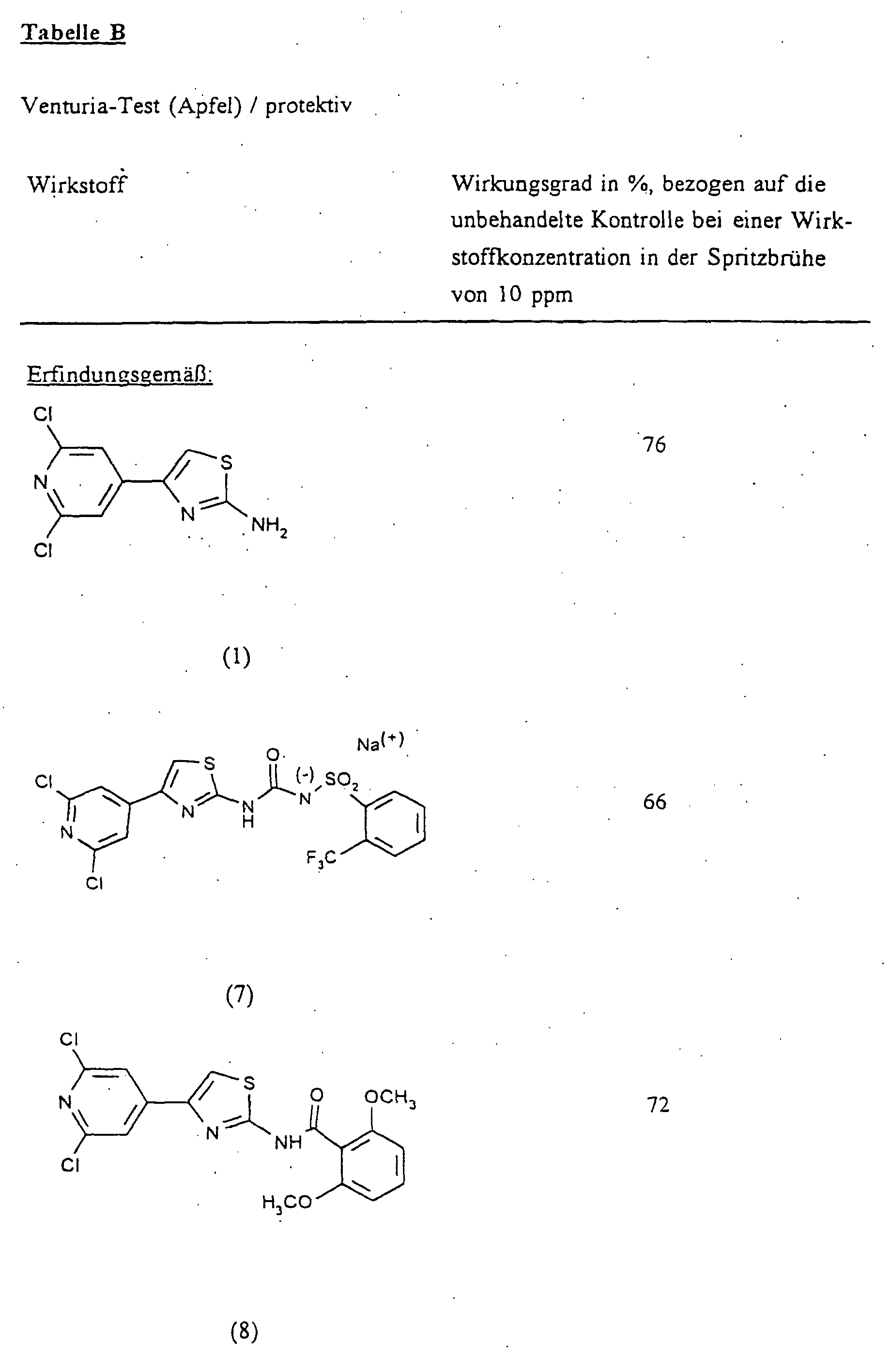

### Beispiel C

### Erysiphe-Test (Gerste) / protektiv

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

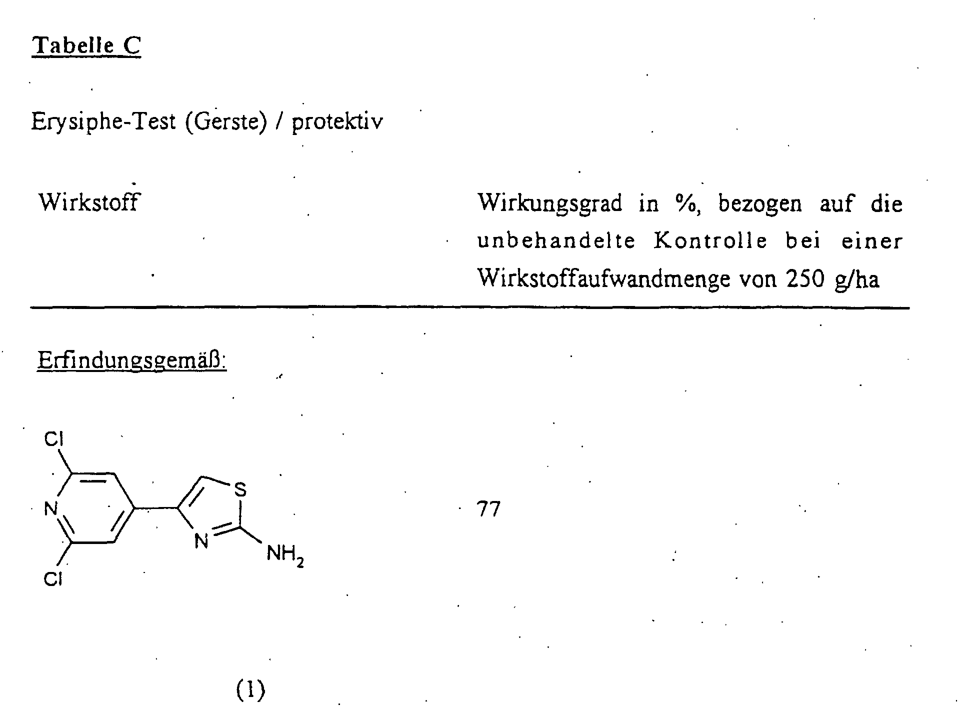

### Beispiel D

### Erysiphe-Test (Gerste) / kurativ

| | |
|---|---|
| Lösungsmittel | 10 Gewichtsteile N-Methyl-pyrrolidon |
| Emulgator | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen..

7 Tage nach der Inokulation erfolgt die Auswertung. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

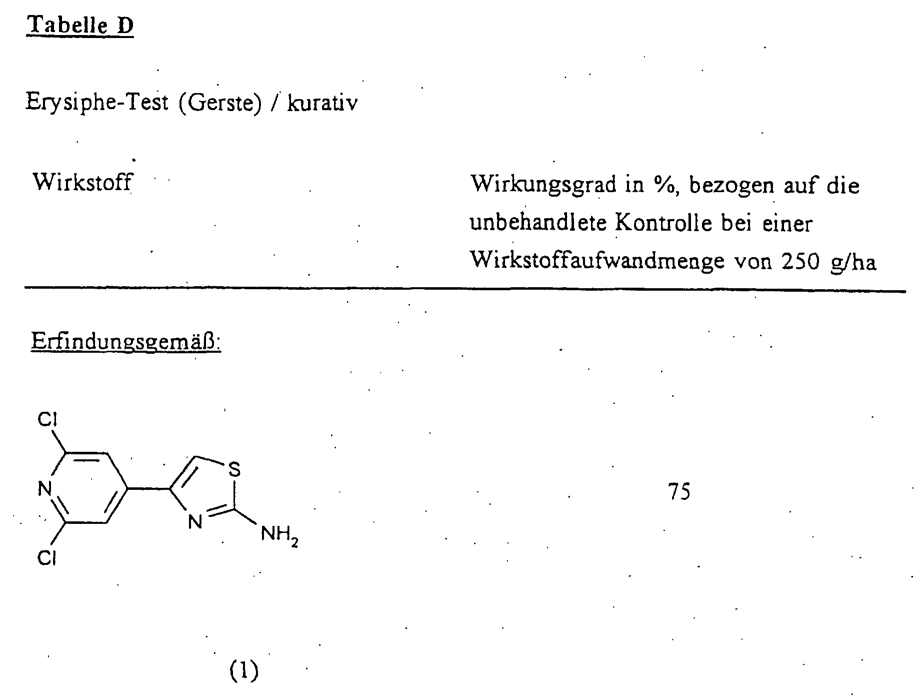

### Beispiel E

### Pyricularia-Test (Reis) / protektiv

| | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe 4 Tage nach dem Antrocknen des Spritzbelages werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

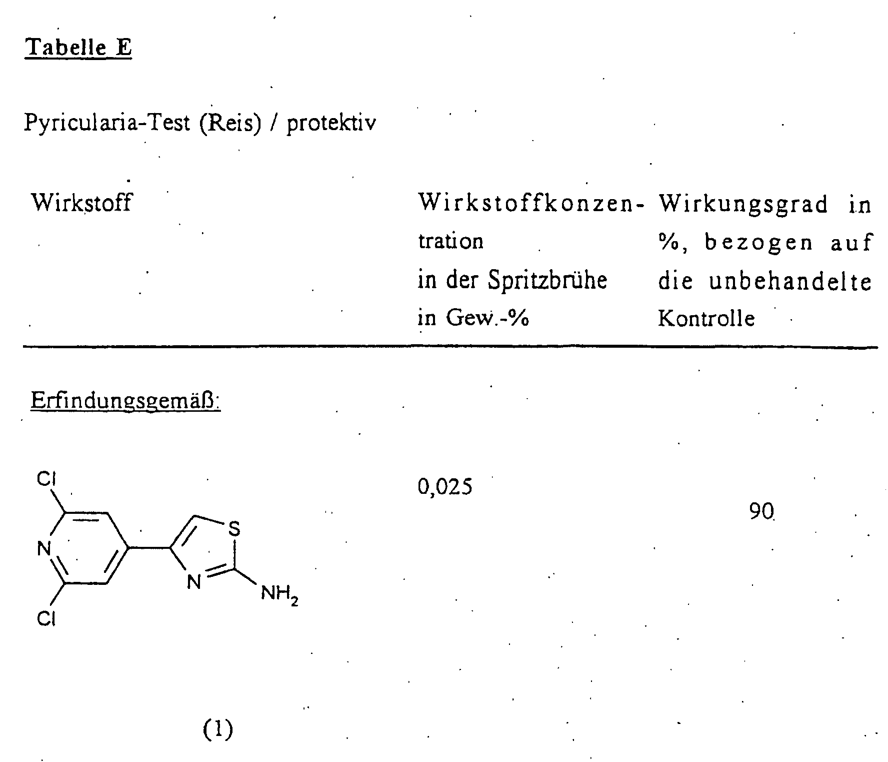

### Beispiel F

### Pyricularia-Test (Reis) / systemisch

| | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100% bis zur Auswertung.

4 Tage nach der Inokulation erfolgt die Auswertung des Krankheitsbefalls. Dabei bedeutet 0 % einen Wirkungsgrad, der demjenigen der Kontrolle entspricht, während ein Wirkungsgrad von 100 % bedeutet, daß kein Befall beobachtet wird.

Wirkstoffe, Wirkstoffkonzentrationen und Versuchsergebnisse gehen aus der folgenden Tabelle hervor.

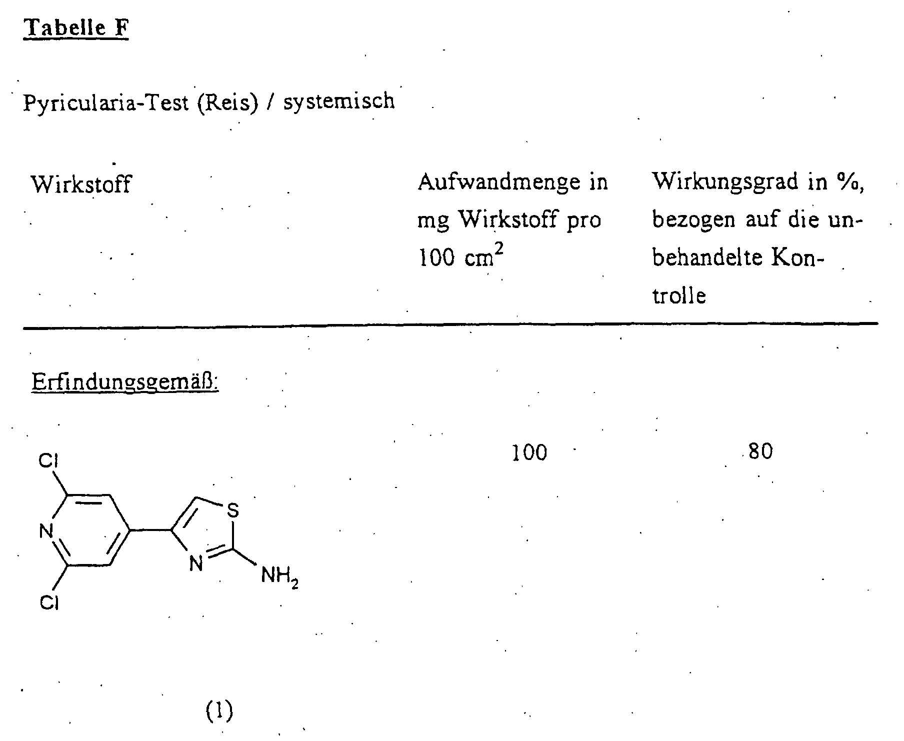

## Patentansprüche

1. Pyridyl-thiazole der Formel 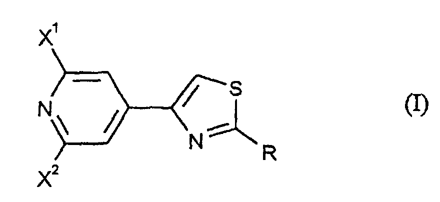 in welcher
R für geradkettiges oder verzweigtes Alkyl mit 1 bis 6 Kohlenstoffatomen sowie für die Gruppen 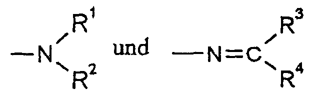 steht, worin
R¹ für Wasserstoff oder -CO-R⁵ steht,
R² für Wasserstoff oder -CO-R⁶ steht,
R³ für Wasserstoff oder geradkettiges oder verzweigtes Alkyl mit 1 bis 4 Kohlenstoffatomen steht,
R⁴ für Alkoxy mit 1 bis 4 Kohlenstoffatomen oder für Dialkylamino mit 1 bis 4 Kohlenstoffatomen in jeder Alkylgruppe steht und
R⁵ und R⁶ unabhängig voneinander für geradkettiges oder verzweigtes Alkyl mit 1 bis 6 Kohlenstoffatomen, Alkoxycarbonyl mit 1 bis 4 Kohlenstoffatomen in der Alkoxygruppe, Alkylamino mit 1 bis 6 Kohlenstoffatomen, Aryl mit 6 bis 10 Kohlenstoffatomen, Arylamino mit 6 bis 10 Kohlenstoffatomen oder Arylsulfonylamino mit 6 bis 10 Kohlenstoffatomen stehen, wobei jeder der drei zuletzt genannten Reste im Arylteil einfach bis dreifach, gleichartig oder verschieden substituiert sein kann durch Halogen, Cyano, Nitro, Formyl, Carboxyl, Carbamoyl, Alkyl mit 1 bis 6 Kohlenstoffatomen, Alkoxy mit 1 bis 6 Kohlenstoffatomen, Alkylthio mit 1 bis 6 Kohlenstoffatomen, Alkylsulfinyl mit 1 bis 4 Kohlenstoffatomen, Alkylsulfonyl mit 1 bis 4 Kohlenstoffatomen, Halogenalkyl mit 1 bis 4 Kohlenstoffatomen und 1 bis 5 Fluor-, Chlor- und/oder Bromatomen, Halogenalkoxy mit 1 bis 4 Kohlenstoffatomen und 1 bis 5 Fluor-, Chlor- und/oder Bromatomen, Halogenalkylthio mit 1 bis 4 Kohlenstoffatomen und 1 bis 5 Fluor-, Chlor- und/oder Bromatomen, Halogenalkylsulfinyl mit 1 bis 4 Kohlenstoffatomen und 1 bis 5 Fluor-, Chlor- und/oder Bromatomen, Halogenalkylsulfonyl mit 1 bis 4 Kohlenstoffatomen und 1 bis 5 Fluor-, Chlor- und/oder Bromatomen, Alkoxycarbonyl mit 1 bis 4 Kohlenstoffatomen im Alkoxyteil, Hydroximinoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil und/oder durch zweifach verknüpftes Alkandiyl mit 3 oder 4 Kohlenstoffatomen, in dem ein oder zwei (nicht benachbarte) Kohlenstoffatome durch Sauerstoff ersetzt sein können und in dem der Alkandiylteil einfach bis vierfach, gleichartig oder verschieden substituiert sein kann durch Halogen, Alkyl mit 1 bis 4 Kohlenstoffatomen und/oder Halogenalkyl mit 1 oder 2 Kohlenstoffatomen und 1 bis 3 Fluor-, Chlor- und/oder Bromatomen,
oder
R¹ und R² gemeinsam mit dem Stickstoffatom, an das sie gebunden sind, für ein cyclisches Imid der Formel 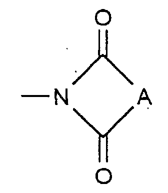 stehen, worin
A für Alkandiyl mit 2 oder 3 Kohlenstoffatomen oder Alkendiyl mit 2 oder 3 Kohlenstoffatomen steht, wobei die genannten Reste jeweils einfach bis vierfach, gleichartig oder verschieden substituiert sein können durch Halogen, Alkyl mit 1 bis 4 Kohlenstoffatomen, Alkoxy mit 1 bis 4 Kohlenstoffatomen, Halogenalkyl mit 1 bis 4 Kohlenstoffatomen und 1 bis 5 Fluor-, Chlor- und/oder Bromatomen und/oder durch Halogenalkoxy mit 1 bis 4 Kohlenstoffatomen und 1 bis 5 Fluor-, Chlor- und/oder Bromatomen,
X¹ für Wasserstoff, Fluor, Chlor oder Brom steht
und
X² für Fluor, Chlor oder Brom steht,
sowie deren Salze und Säureaddukte.

2. Verfahren zur Herstellung von Pyridyl-thiazolen der Formel (I) gemäß Anspruch 1 sowie von deren Salzen und Säureaddukten, **dadurch gekennzeichnet, daß** man
a) Halogenacetyl-pyridine der Formel 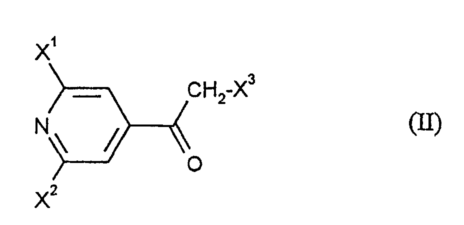 in welcher
X¹ und X² die in Anspruch 1 angegebenen Bedeutungen haben und
X³ für Halogen steht,
mit Thioverbindungen der Formel 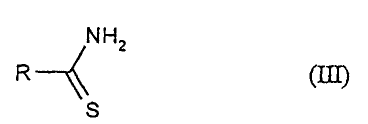 in welcher
R die in Anspruch 1 angegebene Bedeutung hat,
gegebenenfalls in Gegenwart eines Verdünnungsmittels und gegebenenfalls in Gegenwart eines Säurebindemittels umsetzt,
oder
b) Aminothiazole der Formel 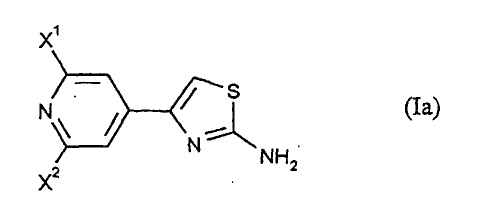 in welcher
X¹ und X² die in Anspruch 1 angegebenen Bedeutungen haben,
entweder
α) mit Carbonsäureanhydriden der Formeln 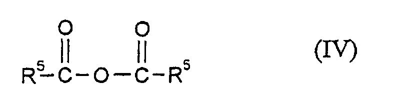 in welcher
R⁵ die in Anspruch 1 angegebene Bedeutung hat,
oder 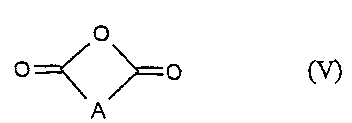 in welcher
A die in Anspruch 1 angegebene Bedeutung hat,
oder
β) mit Carbonsäurehalogeniden der Formel 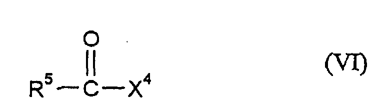 in welcher
R⁵ die in Anspruch 1 angegebene Bedeutung hat und
X⁴ für Halogen steht,
oder
γ) mit Isocyanaten der Formel
R⁷-N=C=O (VII)
in welcher
R⁷ für geradkettiges oder verzweigtes Alkyl mit 1 bis 6 Kohlenstoffatomen, Aryl mit 6 bis 10 Kohlenstoffatomen oder Arylsulfonyl mit 6 bis 10 Kohlenstoffatomen steht,
wobei jeder der zwei zuletzt genannten Reste im Arylteil einfach bis dreifach, gleichartig oder verschieden substituiert sein kann durch Halogen, Cyano, Nitro, Formyl, Carboxyl, Carbamoyl, Alkyl mit 1 bis 6 Kohlenstoffatomen, Alkoxy mit 1 bis 6 Kohlenstoffatomen, Alkylthio mit 1 bis 6 Kohlenstoffatomen, Alkylsulfinyl mit 1 bis 4 Kohlenstoffatomen, Alkylsulfonyl mit 1 bis 4 Kohlenstoffatomen, Halogenalkyl mit 1 bis 4 Kohlenstoffatomen und 1 bis 5 Fluor-, Chlor- und/oder Bromatomen, Halogenalkoxy mit 1 bis 4 Kohlenstoffatomen und 1 bis 5 Fluor-, Chlor- und/oder Bromatomen, Halogenalkylthio mit 1 bis 4 Kohlenstoffatomen und 1 bis 5 Fluor-, Chlor- und/oder Bromatomen, Halogenalkylsulfinyl mit 1 bis 4 Kohlenstoffatomen und 1 bis 5 Fluor-, Chlor- und/oder Bromatomen, Halogenalkylsulfonyl mit 1 bis 4 Kohlenstoffatomen und 1 bis 5 Fluor-, Chlor- und/oder Bromatomen, Alkoxycarbonyl mit 1 bis 4 Kohlenstoffatomen im Alkoxyteil, Hydroximinoalkyl mit 1 bis 4 Kohlenstoffatomen im Alkylteil, Alkoximinoalkyl mit 1 bis 4 Kohlenstoffatomen im Alkoxyteil und 1 bis 4 Kohlenstoffatomen im Alkylteil und/oder durch zweifach verknüpftes Alkandiyl mit 3 oder 4 Kohlenstoffatomen, in dem ein oder zwei (nicht benachbarte) Kohlenstoffatome durch Sauerstoff ersetzt sein können und in dem der Alkandiylteil einfach bis vierfach, gleichartig oder verschieden substituiert sein kann durch Halogen, Alkyl mit 1 bis 4 Kohlenstoffatomen und/oder Halogenalkyl mit 1 oder 2 Kohlenstoffatomen und 1 bis 3 Fluor-, Chlor- und/oder Bromatomen,
oder
δ) mit Acetalen der Formel 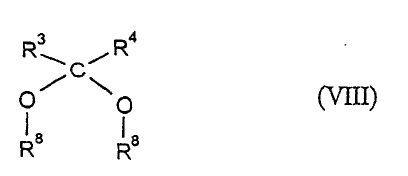 in welcher
R³ und R⁴ die in Anspruch 1 angegebenen Bedeutungen haben und
R⁸ für Methyl oder Ethyl steht,
gegebenenfalls in Gegenwart eines Verdünnungsmittels, gegebenenfalls eines Katalysators und gegebenenfalls in Gegenwart eines Säurebindemittels umsetzt,
und gegebenenfalls anschließend die so erhaltenen Verbindungen der Formel (I) mit einer Säure oder Base umsetzt.

3. Mittel zum Schutz von Pflanzen gegen Befall durch unerwünschte Mikroorganismen, **gekennzeichnet durch** einen Gehalt an mindestens einem Pyridyl-thiazol der Formel (I) gemäß Anspruch 1 bzw. eines Salzes oder Säureadduktes eines Pyridyl-thiazols der Formel (I).

4. Verwendung von Pyridyl-thiazolen der Formel (I) gemäß Anspruch 1 bzw. von deren Salzen oder Säureaddukten zum Schutz von Pflanzen gegen Befall durch unerwünschte Mikroorganismen.

5. Verfahren zum Schutz von Pflanzen gegen Befall durch unerwünschte Mikroorganismen, **dadurch gekennzeichnet, daß** man Pyridyl-thiazole der Formel (I) gemäß Anspruch 1 bzw. deren Salze oder Säureaddukte auf die Mikroorganismen und/oder deren Lebensraum ausbringt.

6. Verfahren zur Herstellung von Mitteln zum Schutz von Pflanzen gegen Befall durch unerwünschte Mikroorganismen, **dadurch gekennzeichnet, daß** man Pyridyl-thiazole der Formel (I) gemäß Anspruch 1 bzw. deren Salze oder Säureaddukte mit Streckmitteln und/oder oberflächenaktiven Stoffen vermischt.

7. Halogenacetyl-pyridine der Formel 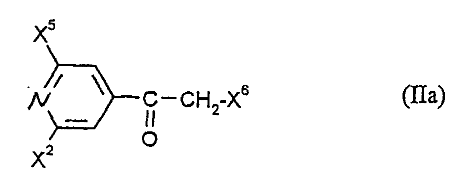 in welcher
X² für Halogen steht,
X⁵ für Halogen steht und
X⁶ für Chlor, Brom oder Iod steht.

8. Verfahren zur Herstellung von Halogenacetyl-pyridinen der Formel (IIa) gemäß Anspruch 7, **dadurch gekennzeichnet, daß** man
c) Acetylpyridine der Formel 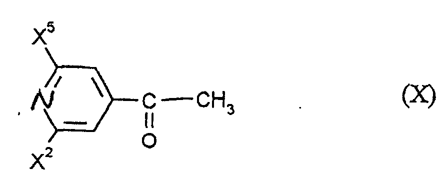 in welcher
X² und X⁵ die in Anspruch 7 angegebenen Bedeutungen haben,
mit Halogenierungsmitteln gegebenenfalls in Gegenwart eines Verdünnungsmittels und gegebenenfalls in Gegenwart eines Katalysators umsetzt.

9. Acetylpyridine der Formel 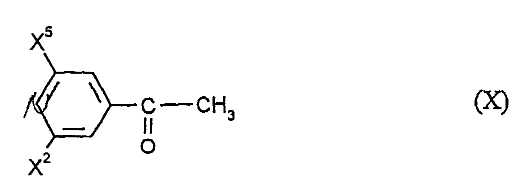 in welcher
X² für Halogen steht und
X⁵ für Halogen steht.

10. Verfahren zur Herstellung von Acetylpyridinen der Formel (X) gemäß Anspruch 9, **dadurch gekennzeichnet, daß** man
d) Malonester-Derivate der Formel 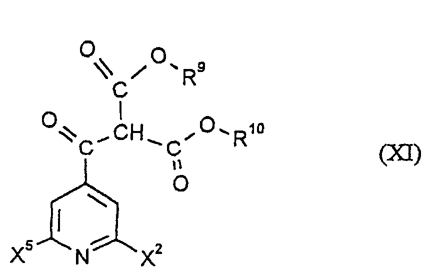 in welcher
X² und X⁵ die in Anspruch 9 angegebenen Bedeutungen haben,
R⁹ für Alkyl steht und
R¹⁰ für Alkyl steht,
mit Wasser gegebenenfalls in Gegenwart eines Verdünnungsmittels umsetzt.

## Claims

1. Pyridyl-thiazoles of the formula 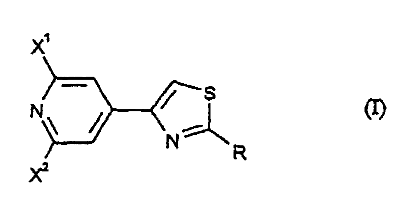 in which
R represents straight-chain or branched alkyl having 1 to 6 carbon atoms or the groups 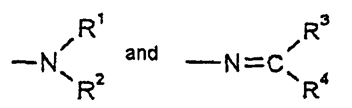 wherein
R¹ represents hydrogen or -CO-R⁵,
R² represents hydrogen or -CO-R⁶,
R³ represents hydrogen or straight-chain or branched alkyl having 1 to 4 carbon atoms,
R⁴ represents alkoxy having 1 to 4 carbon atoms, or represents dialkylamino having 1 to 4 carbon atoms in each alkyl group, and
R⁵ and R⁶ independently of one another preferably represent straight-chain or branched alkyl having 1 to 6 carbon atoms, alkoxycarbonyl having 1 to 4 carbon atoms in the alkoxy group, alkylamino having 1 to 6 carbon atoms, aryl having 6 to 10 carbon atoms, arylamino having 6 to 10 carbon atoms or arylsulphonylamino having 6 to 10 carbon atoms, it being possible for each of the three latter radicals to be mono- to trisubstituted in the aryl part in an identical or different manner by halogen, cyano, nitro, formyl, carboxyl, carbamoyl, alkyl having 1 to 6 carbon atoms, alkoxy having 1 to 6 carbon atoms, alkylthio having 1 to 6 carbon atoms, alkylsulphinyl having 1 to 4 carbon atoms, alkylsulphonyl having 1 to 4 carbon atoms, halogenoalkyl having 1 to 4 carbon atoms and 1 to 5 fluorine, chlorine and/or bromine atoms, halogenoalkoxy having 1 to 4 carbon atoms and 1 to 5 fluorine, chlorine and/or bromine atoms, halogenoalkylthio having 1 to 4 carbon atoms and 1 to 5 fluorine, chlorine and/or bromine atoms, halogenoalkylsulphinyl having 1 to 4 carbon atoms and 1 to 5 fluorine, chlorine and/or bromine atoms, halogenoalkylsulphonyl having 1 to 4 carbon atoms and 1 to 5 fluorine, chlorine and/or bromine atoms, alkoxycarbonyl having 1 to 4 carbon atoms in the alkoxy part, hydroximinoalkyl having 1 to 4 carbon atoms in the alkyl part, alkoximinoalkyl having 1 to 4 carbon atoms in the alkoxy part and 1 to 4 carbon atoms in the alkyl part and/or divalent alkanediyl having 3 or 4 carbon atoms, in which one or two (non-adjacent) carbon atoms can be replaced by oxygen and in which the alkanediyl part can be mono- to tetrasubstituted in an identical or different manner by halogen, alkyl having 1 to 4 carbon atoms and/or halogenoalkyl having 1 or 2 carbon atoms and 1 to 3 fluorine, chlorine and/or bromine atoms,
or
R¹and R², together with the nitrogen atom to which they are bonded, represent a cyclic imide of the formula 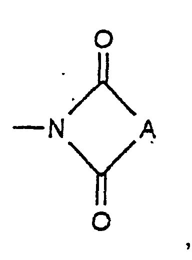 wherein
A represents alkanediyl having 2 or 3 carbon atoms or alkenediyl having 2 or 3 carbon atoms, it being possible for the radicals mentioned in each case to be mono- to tetrasubstituted in an identical or different manner by halogen, alkyl having 1 to 4 carbon atoms, alkoxy having 1 to 4 carbon atoms, halogenoalkyl having 1 to 4 carbon atoms and 1 to 5 fluorine, chlorine and/or bromine atoms and/or halogenoalkoxy having 1 to 4 carbon atoms and 1 to 5 fluorine, chlorine and/or bromine atoms,
X¹ represents hydrogen, fluorine, chlorine or bromine,
and
X² represents fluorine, chlorine or bromine,
and salts and acid adducts thereof.

2. Process for the preparation of pyridyl-thiazoles of the formula (I) according to Claim 1 and of salts and acid adducts thereof, **characterized in that**
a) halogenoacetyl-pyridines of the formula 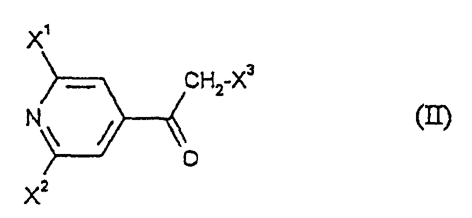 in which
X¹ and X² are as defined in Claim 1 and
X³ represents halogen,
are reacted with thio compounds of the formula 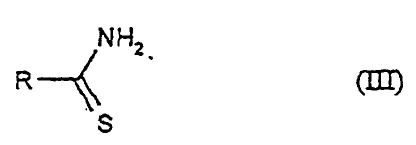 in which
R is as defined in Claim 1,
if appropriate in the presence of a diluent and if appropriate in the presence of an acid-binding agent,
or
b) aminothiazoles of the formula 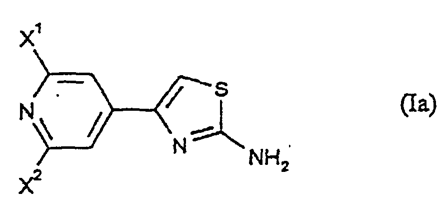 in which
X¹ and X² are as defined in Claim 1,
are reacted either
α) with carboxylic anhydrides of the formulae 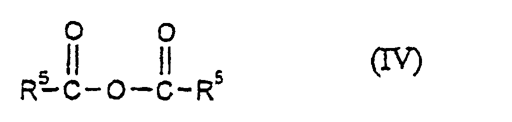 in which
R⁵ is as defined in Claim 1
or 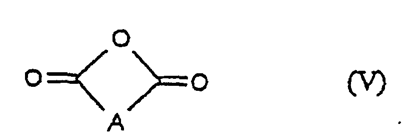 in which
A is as defined in Claim 1,
or
β) with carboxylic halides of the formula 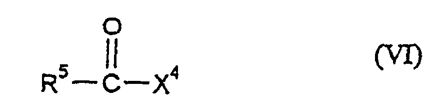 in which
R⁵ is as defined in Claim 1 and
X⁴ represents halogen,
or
γ) with isocyanates of the formula
R⁷-N=C=O (VII)
in which
R⁷ represents straight-chain or branched alkyl having 1 to 6 carbon atoms, aryl having 6 to 10 carbon atoms or arylsulphonyl having 6 to 10 carbon atoms, it being possible for each of the two latter radicals to be mono- to trisubstituted in the aryl part in an identical or different manner by halogen, cyano, nitro, formyl, carboxyl, carbamoyl, alkyl having 1 to 6 carbon atoms, alkoxy having 1 to 6 carbon atoms, alkylthio having 1 to 6 carbon atoms, alkylsulphinyl having 1 to 4 carbon atoms, alkylsulphonyl having 1 to 4 carbon atoms, halogenoalkyl having 1 to 4 carbon atoms and 1 to 5 fluorine, chlorine and/or bromine atoms, halogenoalkoxy having 1 to 4 carbon atoms and 1 to 5 fluorine, chlorine and/or bromine atoms, halogenoalkylthio having 1 to 4 carbon atoms and 1 to 5 fluorine, chlorine and/or bromine atoms, halogenoalkylsulphinyl having 1 to 4 carbon atoms and 1 to 5 fluorine, chlorine and/or bromine atoms, halogenoalkylsulphonyl having 1 to 4 carbon atoms and 1 to 5 fluorine, chlorine and/or bromine atoms, alkoxycarbonyl having 1 to 4 carbon atoms in the alkoxy part, hydroximinoalkyl having 1 to 4 carbon atoms in the alkyl part, alkoximinoalkyl having 1 to 4 carbon atoms in the alkoxy part and 1 to 4 carbon atoms in the alkyl part and/or divalent alkanediyl having 3 or 4 carbon atoms, in which one or two (non-adjacent) carbon atoms can be replaced by oxygen and in which the alkanediyl part can be mono- to tetrasubstituted in an identical or different manner by halogen, alkyl having 1 to 4 carbon atoms and/or halogenoalkyl having 1 or 2 carbon atoms and 1 to 3 fluorine, chlorine and/or bromine atoms,
or
δ) with acetals of the formula 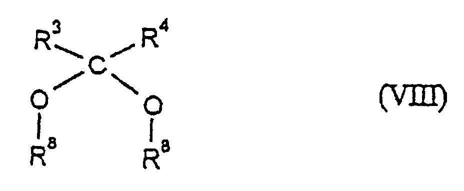 in which
R³ and R⁴ are as defined in Claim 1 and
R⁸ represents methyl or ethyl,
if appropriate in the presence of a diluent, if appropriate in the presence of a catalyst and if appropriate in the presence of an acid-binding agent,
and, if appropriate, the compounds of the formula (I) thus obtained are then reacted with an acid or base.

3. Composition for protecting plants against attack by undesirable microorganisms, **characterized by** a content of at least one pyridylthiazole of the formula (I) according to Claim 1 or of a salt or acid adduct of a pyridyl-thiazole of the formula (I).

4. Use of pyridyl-thiazoles of the formula (I) according to Claim 1 or of salts or acid adducts thereof for protecting plants against attack by undesirable microorganisms.

5. Method for protecting plants against attack by undesirable microorganisms, **characterized in that** pyridyl-thiazoles of the formula (I) according to Claim 1 or salts or acid adducts thereof are applied to the microorganisms and/or their environment.

6. Process for the preparation of compositions for protecting plants against attack by undesirable microorganisms, **characterized in that** pyridyl-thiazoles of the formula (I) according to Claim 1 or salts or acid adducts thereof are mixed with extenders and/or surface-active substances.

7. Halogenoacetyl-pyridines of the formula 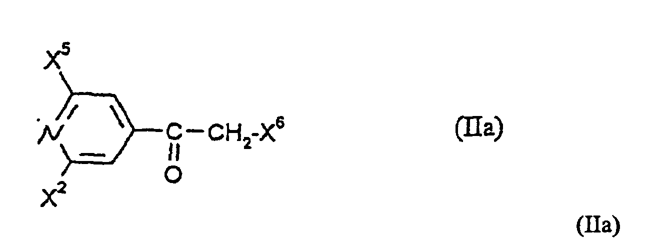 in which
X² represents halogen,
X⁵ represents halogen and
X⁶ represents chlorine, bromine or iodine.

8. Process for the preparation of halogenoacetyl-pyridines of the formula (IIa) according to Claim 7, **characterized in that**
c) acetylpyridines of the formula 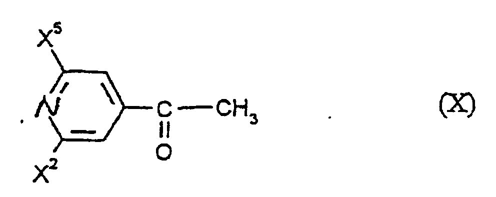 in which
X² and X⁵ are as defined in Claim 7,
are reacted with halogenating agents, if appropriate in the presence of a diluent and if appropriate in the presence of a catalyst.

9. Acetylpyridines of the formula 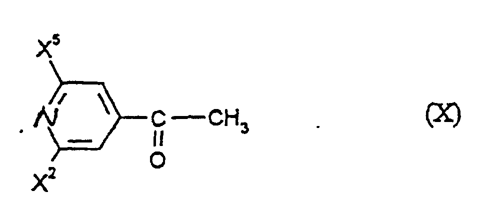 in which
X² represents halogen and
X⁵ represents halogen.

10. Process for the preparation of acetylpyridines of the formula (X) according to Claim 9, **characterized in that**
d) malonic ester derivatives of the formula 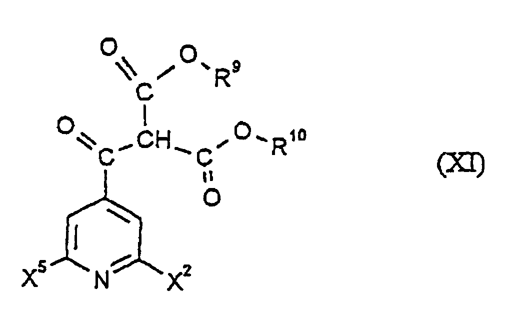 in which
X² and X⁵ are as defined in Claim 9,
R⁹ represents alkyl and
R¹⁰ represents alkyl,
are reacted with water, if appropriate in the presence of a diluent.

## Revendications

1. Pyridyl-thiazoles de formule 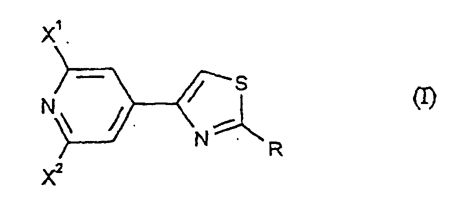 dans laquelle
R représente un reste alkyle linéaire ou ramifié ayant 1 à 6 atomes de carbone, ainsi que les groupes 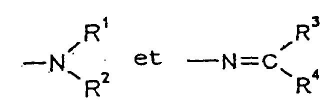 dans lesquels
R¹ est l'hydrogène ou un groupe -CO-R⁵,
R² est l'hydrogène ou un groupe -CO-R⁶,
R³ est l'hydrogène ou un reste alkyle linéaire ou ramifié ayant 1 à 4 atomes de carbone,
R⁴ est un reste alkoxy ayant 1 à 4 atomes de carbone ou un reste dialkylamino ayant 1 à 4 atomes de carbone dans chaque groupe alkyle, et
R⁵ et R⁶ représentent indépendamment l'un de l'autre un reste alkyle linéaire ou ramifié ayant 1 à 6 atomes de carbone, alkoxycarbonyle ayant 1 à 4 atomes de carbone dans le groupe alkoxy, alkylamino ayant 1 à 6 atomes de carbone, aryle ayant 6 à 10 atomes de carbone, arylamino ayant 6 à 10 atomes de carbone ou arylsulfonylamino ayant 6 à 10 atomes de carbone, chacun des trois restes mentionnés en dernier lieu pouvant être substitué dans la partie aryle une à trois fois identiques ou différentes par un radical halogéno, cyano, nitro, formyle, carboxyle, carbamoyle, alkyle ayant 1 à 6 atomes de carbone, alkoxy ayant 1 à 6 atomes de carbone, alkylthio ayant 1 à 6 atomes de carbone, alkylsulfinyle ayant 1 à 4 atomes de carbone, alkylsulfonyle ayant 1 à 4 atomes de carbone, halogénalkyle ayant 1 à 4 atomes de carbone et 1 à 5 atomes de fluor, de chlore et/ou de brome, halogénalkoxy ayant 1 à 4 atomes de carbone et 1 à 5 atomes de fluor, de chlore et/ou de brome, halogénalkylthio ayant 1 à 4 atomes de carbone et 1 à 5 atomes de fluor, de chlore et/ou de brome, halogénalkylsulfinyle ayant 1 à 4 atomes de carbone et 1 à 5 atomes de fluor, de chlore et/ou de brome, halogénalkylsulfonyle ayant 1 à 4 atomes de carbone et 1 à 5 atomes de fluor, de chlore et/ou de brome, alkoxycarbonyle ayant 1 à 4 atomes de carbone dans la partie alkoxy, hydroxyminoalkyle ayant 1 à 4 atomes de carbone dans la partie alkyle, alkoxyminoalkyle ayant 1 à 4 atomes de carbone dans la partie alkoxy et 1 à 4 atomes de carbone dans la partie alkyle, et/ou par un radical alcanediyle à deux liaisons ayant 3 ou 4 atomes de carbone, dans lequel 1 ou 2 atomes de carbone (non contigus) peuvent être remplacés par de l'oxygène et dans lequel la partie alcanediyle peut être substituée une à quatre fois identiques ou différentes par un radical halogéno, alkyle ayant 1 à 4 atomes de carbone et/ou halogénalkyle ayant 1 ou 2 atomes de carbone et 1 à 3 atomes de fluor, de chlore et/ou de brome,
ou bien
R¹ et R² forment conjointement avec l'atome d'azote auquel ils sont liés un imide cyclique de formule 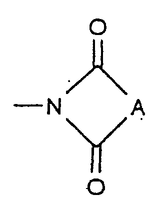 dans laquelle
A représente un reste alcanediyle ayant 2 ou 3 atomes de carbone ou alcéndiyle ayant 2 ou 3 atomes de carbone, les restes mentionnés pouvant chacun être substitué une à quatre fois identiques ou différentes par un radical halogéno, alkyle ayant 1 à 4 atomes de carbone, alkoxy ayant 1 à 4 atomes de carbone, halogénalkyle ayant 1 à 4 atomes de carbone et 1 à 5 atomes de fluor, de chlore et/ou de brome, et/ou par un radical halogénalkoxy ayant 1 à 4 atomes de carbone et 1 à 5 atomes de fluor, de chlore et/ou de brome,
X¹ représente l'hydrogène, le fluor, le chlore ou le brome,
et
X² représente le fluor, le chlore ou le brome,
ainsi que leurs sels et leurs produits d'addition d'acides.

2. Procédé de production de pyridyl-thiazoles de formule (I) suivant la revendication 1 ainsi que de leurs sels et de leurs produits d'addition d'acides, **caractérisé en ce que**
(a) on fait réagir des halogénacétyl-pyridines de formule 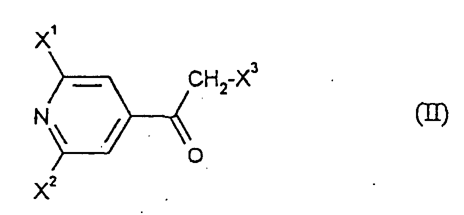 dans laquelle
X¹ et X² ont les définitions indiquées dans la revendication 1 et
X³ représente un halogène,
avec des composés thio de formule 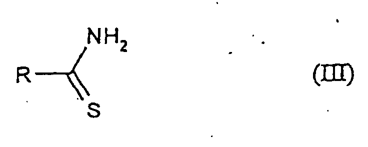 dans laquelle
R a la définition indiquée dans la revendication 1, éventuellement en présence d'un diluant et, le cas échéant, en présence d'un accepteur d'acide,
ou bien
b) on fait réagir des aminothiazoles de formule 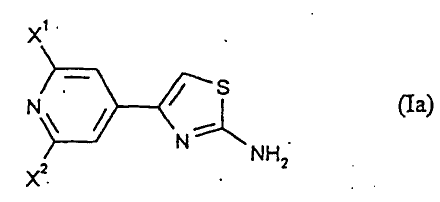 dans laquelle
X¹ et X² ont les définitions indiquées dans la revendication 1,
ou bien
α) avec des anhydrides d'acides carboxyliques de formule 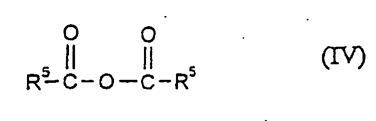 dans laquelle
R⁵ a la définition indiquée dans la revendication 1,
ou 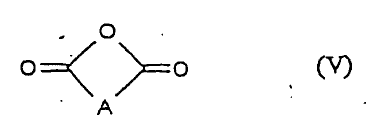 dans laquelle
A a la définition indiquée dans la revendication 1,
ou
β) avec des halogénures d'acides carboxyliques de formule 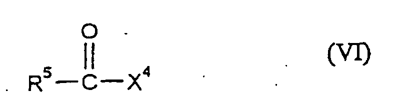 dans laquelle
R⁵ a la définition indiquée dans la revendication 1,
et
X⁴ est un halogène,
ou bien
γ) avec des isocyanates de formule
R⁷-N=C=O (VII)
dans laquelle
R⁷ est un reste alkyle linéaire ou ramifié ayant 1 à 6 atomes de carbone, un reste aryle ayant 6 à 10 atomes de carbone ou un reste arylsulfonyle ayant 6 à 10 atomes de carbone, chacun des deux restes mentionnés en dernier lieu pouvant être substitué dans la partie aryle une à trois fois identiques ou différentes par un radical halogéno, cyano, nitro, formyle, carboxyle, carbamoyle, alkyle ayant 1 à 6 atomes de carbone, alkoxy ayant 1 à 6 atomes de carbone, alkylthio ayant 1 à 6 atomes de carbone, alkylsulfinyle ayant 1 à 4 atomes de carbone, alkylsulfonyle ayant 1 à 4 atomes de carbone, halogénalkyle ayant 1 à 4 atomes de carbone et 1 à 5 atomes de fluor, de chlore et/ou de brome, halogénalkoxy ayant 1 à 4 atomes de carbone et 1 à 5 atomes de fluor, de chlore et/ou de brome, halogénalkylthio ayant 1 à 4 atomes de carbone et 1 à 5 atomes de fluor, de chlore et/ou de brome, halogénalkylsulfinyle ayant 1 à 4 atomes de carbone et 1 à 5 atomes de fluor, de chlore et/ou de brome, halogénalkylsulfonyle ayant 1 à 4 atomes de carbone et 1 à 5 atomes de fluor, de chlore et/ou de brome, alkoxycarbonyle ayant 1 à 4 atomes de carbone dans la partie alkoxy, hydroxyminoalkyle ayant 1 à 4 atomes de carbone dans la partie alkyle, alkoxyminoalkyle ayant 1 à 4 atomes de carbone dans la partie alkoxy et 1 à 4 atomes de carbone dans la partie alkyle et/ou par un radical alcanediyle à deux liaisons ayant 3 ou 4 atomes de carbone, dans lequel 1 ou 2 atomes de carbone (non contigus) peuvent être remplacés par de l'oxygène et dans lequel la partie alcanediyle peut être substituée une à quatre fois identiques ou différentes par un radical halogéno, alkyle ayant 1 à 4 atomes de carbone et/ou halogénalkyle ayant 1 ou 2 atomes de carbone et 1 à 3 atomes de fluor, de chlore et/ou de brome,
ou bien
δ) avec des acétals de formule 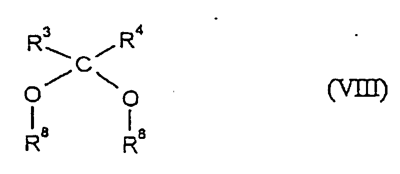 dans laquelle
R³ et R⁴ ont les définitions indiquées dans la revendication 1 et
R⁸ est un reste méthyle ou éthyle,
éventuellement en présence d'un diluant, le cas échéant en présence d'un catalyseur et en présence éventuelle d'un accepteur d'acide,
et on fait ensuite éventuellement réagir les composés de formule (I) ainsi obtenus avec un acide ou avec une base.

3. Composition destinée à protéger les plantes d'une infection par des micro-organismes indésirables, **caractérisée par** une teneur en au moins un pyridyl-thiazole de formule (I) suivant la revendication 1 ou d'un sel ou d'un produit d'addition d'acide d'un pyridyl-thiazole de formule (I)

4. Utilisation de pyridyl-thiazoles de formule (I) suivant la revendication 1 ou de leurs sels ou produits d'addition d'acides pour protéger des plantes d'une infection par des micro-organismes indésirables.

5. Procédé pour protéger des plantes d'une infection par des micro-organismes indésirables, **caractérisé par** l'épandage de pyridyl-thiazoles de formule (I) suivant la revendication 1 ou de leurs sels ou produits d'addition d'acides sur les micro-organismes et/ou sur leur milieu.

6. Procédé de préparation de compositions destinées à protéger les plantes d'une infection par des micro-organismes indésirables, **caractérisé en ce qu'**on mélange des pyridyl-thiazoles de formule (I) suivant la revendication 1 ou leurs sels ou produits d'addition d'acides avec des diluants et/ou des agents tensioactifs.

7. Halogénacétyl-pyridines de formule 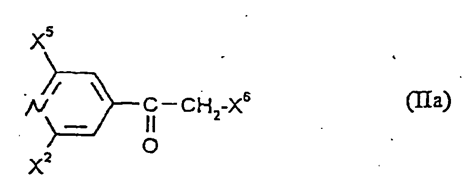 dans laquelle
X² représente un halogène,
X⁵ représente un halogène et
X⁶ représente le chlore, le brome ou l'iode.

8. Procédé de production d'halogénacétyl-pyridines de formule (IIa) suivant la revendication 7, **caractérisé en ce que** :
(c) on fait réagir des acétylpyridines de formule 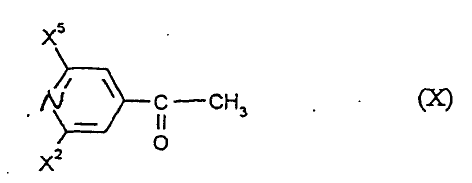 dans laquelle
X² et X⁵ ont les définitions indiquées dans la revendication 7,
avec des agents d'halogénation, éventuellement en présence d'un diluant et le cas échéant en présence d'un catalyseur.

9. Acétylpyridine de formule 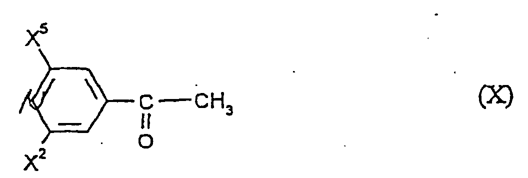 dans laquelle
X² est un halogène et
X⁵ est un halogène.

10. Procédé de production d'acétylpyridines de formule (X) suivant la revendication 9, **caractérisé en ce que** :
(d) on fait réagir des dérivés d'esters maloniques de formule 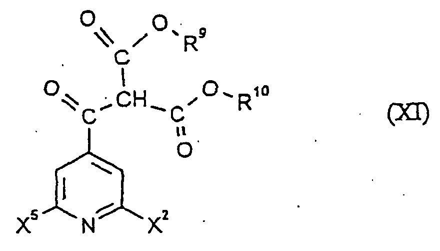 dans laquelle
X² et X⁵ ont les définitions indiquées dans la revendication 9,
R⁹ est un reste alkyle et
R¹⁰ est un reste alkyle,
avec de l'eau en présence éventuelle d'un diluant.
